# EUROPEAN PATENT SPECIFICATION

(11) **EP 1 654 229 B1**
(45) Date of publication and mention of the grant of the patent: **09.05.2007**
(21) Application number: 04749185.7
(22) Date of filing: 26.07.2004
(51) Int. Cl.: C07D 211/28, A61K 31/445, A61P 31/12, A61P 37/02

(54) **PIPERIDINE DERIVATIVES AS CCR5 RECEPTOR MODULATORS**
PIPERIDINDERIVATE ALS CCR5-REZEPTORMODULATOREN
DERIVES DE PIPERIDINE EN TANT QUE MODULATEURS DU RECEPTEUR CCR5

(30) Priority: 31.07.2003 SE 0302155; 03.06.2004 SE 0401420
(43) Date of publication of application: 10.05.2006
(73) Proprietor: AstraZeneca AB, 151 85 Södertälje (SE)
(72) Inventor: BROWN, Dearg, AstraZeneca R & D Alderley, Macclesfield, Cheshire SK10 4TG (GB); OLDFIELD, John, AstraZeneca R & D Alderley, Macclesfield, Cheshire SK10 4TG (GB); TUCKER, Howard, AstraZeneca R & D Alderley, Macclesfield, Cheshire SK10 4TG (GB)
(86) International application number: PCT/SE2004/001149
(87) International publication number: WO 2005/009959

(56) References cited:
- WO-A1-00/38680
- WO-A1-03/042178
- WO-A1-03/042205

## Description

The present invention relates to heterocyclic derivatives having pharmaceutical activity, to processes for preparing such derivatives, to pharmaceutical compositions comprising such derivatives and to the use of such derivatives as active therapeutic agents.

Pharmaceutically active piperidine derivatives are disclosed in WO01/87839, EP-A1-1013276, WO00/08013, WO99/38514, WO99/04794, WO00/76511, WO00/76512, WO00/76513, WO00/76514, WOOO/76972, US 2002/0094989 and Bioorg. Med. Chem. Lett. 13 (2003) 119-123.

Chemokines are chemotactic cytokines that are released by a wide variety of cells to attract macrophages, T cells, eosinophils, basophils and neutrophils to sites of inflammation and also play a rôle in the maturation of cells of the immune system. Chemokines play an important rôle in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C, or α) and Cys-Cys (C-C, or β) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T-cell Expressed and Secreted), eotaxin and the macrophage inflammatory proteins 1α and 1β (MIP-1α and MIP-1β).

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CGR10, CXCR1, CXCR2, CXCR3 and CXCR4. These receptors represent good targets for drug development since agents which modulate these receptors would be useful in the treatment of disorders and diseases such as those mentioned above.

The CCR5 receptor is expressed on T-lymphocytes, monocytes, macrophages, dendritic cells, microglia and other cell types. These detect and respond to several chemokines, principally "regulated on activation normal T-cell expressed and secreted" (RANTES), macrophage inflammatory proteins (MIP) MIP-1α, and MIP-1β and monocyte chemoattractant protein-2 (MCP-2).

This results in the recruitment of cells of the immune system to sites of disease. In many diseases it is the cells expressing CCR5 which contribute, directly or indirectly, to tissue damage. Consequently, inhibiting the recruitment of these cells is beneficial in a wide range of diseases.

CCR5 is also a co-receptor for HIV-1 and other viruses, allowing these viruses to enter cells. Blocking the receptor with a CCR5 antagonist or inducing receptor internalisation with a CCR5 agonist protects cells from viral infection.

The present invention provides a compound of formula (I): 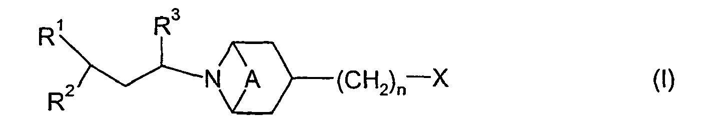 wherein:
A is absent or is (CH₂)₂;
R¹ is C(O)NR¹⁰R¹¹, C(O)₂R¹², NR¹³C(O)R¹⁴, NR¹⁵C(O)NR¹⁶R¹⁷, NR¹⁸C(O)₂R¹⁹, heterocyclyl (for example piperidine, piperazine, pyrrolidine or azetidine), aryl, cycloalkyl or heteroaryl; R¹⁰, R¹³, R¹⁵, R¹⁶ and R¹⁸ are hydrogen or C₁₋₆ alkyl;
R¹¹, R¹², R¹⁴, R¹⁷ and R¹⁹ are C₁₋₈ alkyl (optionally substituted by halo, hydroxy, C₁₋₆ alkoxy, C₁₋₆ haloalkoxy, C₃₋₆ cycloalkyl (optionally substituted by halo), C₅₋₆ cycloalkenyl, S(C₁₋₄ alkyl), S(O)(C₁₋₄ alkyl), S(O)₂(C₁₋₄ alkyl), heteroaryl, aryl, heteroaryloxy or aryloxy), aryl, heteroaryl, C₃₋₇ cycloalkyl (optionally substituted by halo or C₁₋₄ alkyl), C₄₋₇ cycloalkyl fused to a phenyl ring, C₅₋₇ cycloalkenyl, or, heterocyclyl (itself optionally substituted by oxo,
C(O)(C₁₋₆ alkyl), S(O)ₖ(C₁₋₆ alkyl), halo or C₁₋₄ alkyl); or R¹¹, R¹², R¹⁴ and R¹⁷ can also be hydrogen;
or R¹⁰ and R¹¹, and/or R¹⁶ and R¹⁷ may join to form a 4-, 5- or 6-membered ring which optionally includes a nitrogen, oxygen or sulphur atom, said ring being optionally substituted by C₁₋₆ alkyl, S(O)₁(C₁₋₆ alkyl) or C(O)(C₁₋₆ alkyl);
R² is phenyl, heteroaryl or C₃₋₇ cycloalkyl;
R³ is H or C₁₋₄ alkyl;
X is S(O)₂NR⁴R⁵ or NR⁶S(O)₂R⁷;
R⁷ is aryl, heteroaryl, C₁₋₆ alkyl, C₃₋₇ cycloalkyl, heterocyclyl or NR⁸R⁹ wherein NR⁸R⁹ can be cyclized to form a 4-, 5- or 6-membered ring which optionally includes a nitrogen, oxygen or sulphur atom, said ring being optionally substituted by C₁₋₆ alkyl, S(O)ₚ(C₁₋₆ alkyl) or C(O)(C₁₋₆ alkyl);
R⁴ and R⁸ are aryl, heteroaryl, C₁₋₆ alkyl (optionally substituted by hydroxy or C₁₋₆ alkoxy), C₃₋₇ cycloalkyl or heterocyclyl;
R⁵, R⁶ and R⁹ are, independently, hydrogen or C₁₋₆ alkyl;
n is 1, 2 or 3;
aryl, phenyl and heteroaryl moieties are independently optionally substituted by one or more of halo, cyano, nitro, hydroxy, OC(O)NR²⁰R²¹, NR²²R²³, NR²⁴C(O)R²⁵, NR²⁶C(O)NR²⁷R²⁸, S(O)₂NR²⁹R³⁰, NR³¹S(O)₂R³², C(O)NR³³R³⁴, CO₂R³⁶, NR³⁷CO₂R³⁸, S(O)_{q}R³⁹, OS(O)₂R⁴⁹, C₁₋₆ alkyl (optionally mono-substituted by S(O)₂R⁵⁰ or C(O)NR⁵¹R⁵²), C₂₋₆ allcenyl, C₂₋₆ alkynyl, C₃₋₁₀ cycloalkyl, C₁₋₆ haloalkyl, C₁₋₆ alkoxy(C₁₋₆)alkyl, C₁₋₆ alkoxy, C₁₋₆ haloalkoxy, phenyl, phenyl(C₁₋₄)alkyl, phenoxy, phenylthio, phenylS(O), phenyls(O)₂, phenyl(C₁₋₄)alkoxy, heteroaryl, heteroaryl(C₁₋₄)alkyl, heteroaryloxy or heteroaryl(C₁₋₄)alkoxy; wherein any of the immediately foregoing phenyl and heteroaryl moieties are optionally substituted with halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), CF₃ or OCF₃;
unless otherwise stated heterocyclyl is optionally substituted by C₁₋₆ alkyl [optionally substituted by phenyl {which itself optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio, S(O)(C₁₋₄ alkyl) or S(O)₂(C₁₋₄ alkyl)} or heteroaryl {which itself optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}], phenyl {optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio, S(O)(C₁₋₄ alkyl) or S(O)₂(C₁-4 alkyl)}, heteroaryl {optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}, S(O)₂NR⁴⁰R⁴¹, C(O)R⁴², C(O)₂(C₁₋₆ alkyl) (such as tert-butoxycarbonyl), C(O)₂(phenyl(C₁₋₂ alkyl)) (such as benzyloxycarbonyl), C(O)NHR⁴³, S(O)₂R⁴⁴, NHS(O)₂NHR⁴⁵, NHC(O)R⁴⁶, NHC(O)NHR⁴⁷ or NHS(O)₂R⁴⁸, provided none of these last four substituents is linked to a ring nitrogen;
k, 1, p and q are, independently, 0, 1 or 2;
R²⁰, R²², R²⁴, R²⁶, R²⁷ R²⁹, R³¹, R³³, R³⁷, R⁴⁰ and R⁵¹ are, independently, hydrogen or C₁₋₆ alkyl;
R²¹, R²³, R²⁵, R²⁸, R³⁰, R³², R³⁴, R³⁶, R³⁸, R³⁹, R⁴¹, R⁴², R⁴³, R⁴⁴, R⁴⁵, R⁴⁶, R⁴⁷, R⁴⁸, R⁴⁹, R⁵⁰ and R⁵² are, independently, C₁₋₆ alkyl (optionally substituted by halo, hydroxy, C₁₋₆ alkoxy, C₁₋₆ haloalkoxy, C₃₋₆ cycloalkyl, C₅₋₆ cycloalkenyl, S(C₁₋₄ alkyl), S(O)(C₁₋₄ alkyl), S(O)₂(C₁₋₄ alkyl), heteroaryl, phenyl, heteroaryloxy or phenyloxy), C₃₋₇ cycloalkyl, phenyl or heteroaryl; wherein any of the immediately foregoing phenyl and heteroaryl moieties are optionally substituted with halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃;
R²¹, R²³, R²⁵, R²⁸, R³⁰, R³⁴, R³⁵, R³⁶, R⁴¹, R⁴², R⁴³, R⁴⁵, R⁴⁶, R⁴⁷ and R⁵² may additionally be hydrogen;
or a pharmaceutically acceptable salt thereof or a solvate thereof.

Certain compounds of the present invention can exist in different isomeric forms (such as enantiomers, diastereomers, geometric isomers or tautomers). The present invention covers all such isomers and mixtures thereof in all proportions.

Suitable salts include acid addition salts such as a hydrochloride, hydrobromide, phosphate, acetate, fumarate, maleate, tartrate, citrate, oxalate, methanesulphonate or *p-*toluenesulphonate. Further acid addition salts include succinate and malonate.

The compounds of the invention may exist as solvates (such as hydrates) and the present invention covers all such solvates.

Alkyl groups and moieties are straight or branched chain and, for example, comprise one to six (such as one to four) carbon atoms. Alkyl is, for example, methyl, ethyl, n-propyl, iso-propyl, n-butyl, sec-butyl or tert-butyl. Methyl is sometimes abbreviated to Me hereinbelow.

Haloalkyl includes CF₃, and haloalkoxy includes OCF₃.

Fluoroalkyl includes, for example, one to six, such as one to three, fluorine atoms, and comprises, for example, a CF₃ group. Fluoroalkyl is, for example, CF₃ or CH₂CF₃.

Cycloalkyl is, for example, cyclopropyl, cyclopentyl or cyclohexyl (such as cyclohexyl). Cycloalkenyl includes cyclopentenyl.

Heterocyclyl is linked by a ring-carbon or ring-heteroatom (such as a ring-nitrogen), and is, for example, piperidine, piperazine, pyrrolidine, azetidine, tetrahydrofuran, morpholine or thiomorpholine.

Aryl includes phenyl and naphthyl. In one aspect of the invention aryl is phenyl.

Heteroaryl is, for example, an aromatic 5 or 6 membered ring, optionally fused to one or more other rings, comprising at least one heteroatom selected from the group comprising nitrogen, oxygen and sulphur; or an N-oxide thereof, or an S-oxide or S-dioxide thereof. Heteroaryl is, for example, furyl, thienyl (also known as thiophenyl), pyrrolyl, thiazolyl, isothiazolyl, pyrazolyl, oxazolyl, isoxazolyl, imidazolyl, [1,2,4]-triazolyl, tetrazolyl, pyridinyl, pyrimidinyl, pyrazinyl, indolyl, benzo[b]furyl (also known as benzfuryl), benz[b]thienyl (also known as benzthienyl or benzthiophenyl), indazolyl, benzimidazolyl, benztriazolyl, benzoxazolyl, benzthiazolyl, 1,2,3-benzothiadiazolyl, an imidazopyridinyl (such as imidazo[1,2a]pyridinyl), thieno[3,2-b]pyridin-6-yl, 1,2,3-benzoxadiazolyl (also known as benzo[1,2,3]thiadiazolyl), 2,1,3-benzothiadiazolyl, benzofurazan (also known as 2,1,3-benzoxadiazolyl), quinoxalinyl, a pyrazolopyridine (for example 1H-pyrazolo[3,4-b]pyridinyl), quinolinyl, isoquinolinyl, a naphthyridinyl (for example [1,6]naphthyridinyl or [1,8]naphthyridinyl), a benzothiazinyl or dibenzothiophenyl (also known as dibenzothienyl); or an N-oxide thereof, or an S-oxide or S-dioxide thereof. Heteroaryl also includes tetrazolyl.

Aryloxy includes phenoxy.

Heteroaryloxy includes pyridinyloxy and pyrimidinyloxy.

Phenyl(C ₁₋₄ alkyl)alkyl is, for example, benzyl, 1-(phenyl)eth-1-yl or 1-(phenyl)eth-2-yl.

Heteroaryl(C₁₋₄ alkyl)alkyl is, for example, pyridinylmethyl, pyrimidinylmethyl or 1-(pyridinyl)eth-2-yl.

Phenyl(C₁₋₄ alkoxy) is, for example, benzyloxy or plienylCH(CH₃)O.

Heteroaryl(C₁₋₄ alkoxy) is, for example, pyridinylCH₂O, pyrimidinylCH₂O or pyridinylCH(CH₃)O.

Heteroaryl rings can carry various substituents including sulphonyl groups. A sulphonyl group on a heteroaryl ring can be a good leaving group (susceptible to nucleophilic displacement) and examples of such situation are: 2-methanesulphonyl-pyridine and 2- or 4-methanesulphonyl-pyrimidine. The present invention covers compounds including a heteroaryl ring carrying a sulphonyl group which are sufficiently stable (non-reactive) to be isolated using the experimental procedures described.

In one particular aspect the present invention provides a compound of formula (I) wherein: A is absent or is (CH₂)₂; R¹ is C(O)NR¹⁰R¹¹, C(O)₂R¹², NR¹³C(O)R¹⁴, NR¹⁵C(O)NR¹⁶R¹⁷, NR¹⁸C(O)₂R¹⁹, heterocyclyl (for example piperidine, piperazine, pyrrolidine or azetidine), aryl, cycloalkyl or heteroaryl; R¹⁰, R¹³, R¹⁵ , R¹⁶ and R¹⁸ are hydrogen or C₁₋₆ alkyl; R¹¹, R¹², R¹⁴, R¹⁷ and R¹⁹ are C₁₋₈ alkyl (optionally substituted by halo, hydroxy, C₁₋₆ alkoxy, C₁₋₆ haloalkoxy, C₃₋₆ cycloalkyl (optionally substituted by halo), C₅₋₆ cycloalkenyl, S(C₁₋₄ alkyl), S(O)(C₁₋₄ alkyl), S(O)₂(C₁₋₄ alkyl), heteroaryl, aryl, heteroaryloxy or aryloxy), aryl, heteroaryl, C₃₋₇ cycloalkyl (optionally substituted by halo or C₁₋₄ alkyl), C₄₋₇ cycloalkyl fused to a phenyl ring, C₅₋₇ cycloalkenyl, or, heterocyclyl (itself optionally substituted by oxo, C(O)(C₁₋₆ alkyl), S(O)ₖ(C₁₋₆ alkyl), halo or C₁₋₄ alkyl); or R¹¹, R¹², R¹⁴ and R¹⁷ can also be hydrogen; or R¹⁰ and R¹¹, and/or R¹⁶ and R¹⁷ may join to form a 4-, 5- or 6-membered ring which optionally includes a nitrogen, oxygen or sulphur atom, said ring being optionally substituted by C₁₋₆ alkyl, S(O)₁(C₁₋₆ alkyl) or C(O)(C₁₋₆ alkyl); R² is phenyl, heteroaryl or C₃₋₇ cycloalkyl; R³ is H or C₁₋₄ alkyl; X is S(O)₂NR⁴R⁵ or NR⁶S(O)₂R⁷; R⁷ is aryl, heteroaryl, C₁₋₆ alkyl, C₃₋₇ cycloalkyl, heterocyclyl or NR⁸R⁹ wherein NR⁸R⁹ can be cyclized to form a 4-, 5- or 6-membered ring which optionally includes a nitrogen, oxygen or sulphur atom, said ring being optionally substituted by C₁₋₆ alkyl, S(O)ₚ(C₁₋₆ alkyl) or C(O)(C₁₋₆ alkyl); R⁴ and R⁸ are aryl, heteroaryl, C₁₋₆ alkyl, C₃₋₇ cycloalkyl or heterocyclyl; R⁵, R⁶ and R⁹ are, independently, hydrogen or C₁₋₆ alkyl; n is 1, 2 or 3; aryl, phenyl and heteroaryl moieties are independently optionally substituted by one or more of halo, cyano, nitro, hydroxy, OC(O)NR²⁰R²¹, NR²²R²³, NR²⁴C(O)R²⁵, NR²⁶C(O)NR²⁷R²⁸, S(O)₂NR²⁹R³⁰, NR³¹S(O)₂R³², C(O)NR³³R³⁴, COR₂R³⁶, NR³⁷CO₂R³⁸, S(O)_{q}R³⁹, OS(O)₂R⁴⁹, C₁₋₆ alkyl (optionally mono-substituted by S(O)₂R⁵⁰ or C(O)NR⁵¹R⁵²), C₂₋₆ alkenyl, C₂₋₆ alkynyl, C₃₋₁₀ cycloalkyl, C₁₋₆ haloalkyl, C₁₋₆ allcoxy(C₁₋₆)akyl, C₁₋₆ alkoxy, C₁₋₆ haloalkoxy, phenyl, phenyl(C₁₋₄)alkyl, phenoxy, phenylthio, phenylS(O), phenylS(O)₂, phenyl(C₁₋₄)alkoxy, heteroaryl, heteroaryl(C₁₋₄)alkyl, heteroaryloxy or heteroaryl(C₁₋₄)alkoxy; wherein any of the immediately foregoing phenyl and heteroaryl moieties are optionally substituted with halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), CF₃ or OCF₃; unless otherwise stated heterocyclyl is optionally substituted by C₁₋₆ alkyl [optionally substituted by phenyl {which itself optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio, S(O)(C₁₋₄ alkyl) or S(O)₂(C₁₋₄ alkyl)} or heteroaryl {which itself optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}], phenyl {optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}, heteroaryl {optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}, S(O)₂NR⁴⁰R⁴¹, C(O)R⁴², C(O)₂(C₁₋₆ alkyl) (such as tert-butoxycarbonyl), C(O)₂(phenyl(C₁₋₂ alkyl)) (such as benzyloxycarbonyl), C(O)NHR⁴³, S(O)₂R⁴⁴, NHS(O)₂NHR⁴⁵, NHC(O)R⁴⁶, NHC(O)NHR⁴⁷ or NHS(O)₂R⁴⁸, provided none of these last four substituents is linked to a ring nitrogen; k, 1, p and q are, independently, 0, 1 or 2; R²⁰, R²², R²⁴, R²⁶, R²⁷, R²⁹, R³¹, R³³, R³⁷, R⁴⁰ and R⁵¹ are, independently, hydrogen or C₁₋₆ alkyl; R²¹, R²³, R²⁵, R²⁸, R³⁰, R³², R³⁴, R³⁶, R³⁸, R³⁹, R⁴¹, R⁴², R⁴³, R⁴⁴, R⁴⁵, R⁴⁶, R⁴⁷, R⁴⁸, R⁴⁹, R⁵⁰ and R⁵² are, independently, C₁₋₆ alkyl (optionally substituted by halo, hydroxy, C₁₋₆ alkoxy, C₁₋₆ haloalkoxy, C₃₋₆ cycloalkyl, C₅₋₆ cycloalkenyl, S(C₁₋₄ alkyl), S(O)(C₁₋₄ alkyl), S(O)₂(C₁₋₄ alkyl), heteroaryl, phenyl, heteroaryloxy or phenyloxy), C₃₋₇ cycloalkyl, phenyl or heteroaryl; wherein any of the immediately foregoing phenyl and heteroaryl moieties are optionally substituted with halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂C₁₋₄ alkyl), C(O)(C₁₋₄alkyl), CF₃ or OCF₃; R²¹, R²³, R²⁵, R²⁸, R³⁰, R³⁴, R³⁵, R³⁶, R⁴¹, R⁴², R⁴³, R⁴⁵, R⁴⁶, R⁴⁷ and R⁵² may additionally be hydrogen; or a pharmaceutically acceptable salt thereof or a solvate thereof.

In another aspect the present invention provides a compound wherein, unless specified otherwise aryl, phenyl and heteroaryl moieties are independently optionally substituted by one or more of halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), CO₂H, CO₂(C₁₋₄ alkyl), NHC(O)(C₁₋₄ alkyl), NHS(O)₂(C₁₋₄ alkyl), CF₃, CHF₂, CH₂F, CH₂CF₃, OCF₃ or tetrazolyl (optionally substituted by C₁₋₄ alkyl).

In a further aspect of the invention heteroaryl is pyrrolyl, thienyl, imidazolyl, thiazolyl, isoxazolyl, pyridinyl, pyrimidinyl, pyrazinyl or quinolinyl.

In another aspect of the invention R¹⁰, R¹³, R¹⁵, R¹⁶ and R¹⁸ are hydrogen or C₁₋₄ alkyl (for example methyl). In yet another aspect R¹⁰, R¹³, R¹⁵, R¹⁶ and R¹⁸ are hydrogen.

In a further aspect of the invention R¹¹, R¹² , R¹⁴ , R¹⁷, R¹⁸ and R¹⁹ are C₁₋₈ alkyl (optionally substituted by halo, C₁₋₆ alkoxy, C₁₋₆ haloalkoxy, C₃₋₆ cycloalkyl (optionally substituted by halo), C₅₋₆ cycloalkenyl, S(O)₂(C₁₋₄ alkyl), heteroaryl, phenyl, heteroaryloxy or aryloxy (for example phenoxy)), phenyl, heteroaryl, C₃₋₇ cycloalkyl (optionally substituted by halo or C₁₋₄ alkyl), C₄₋₇ cycloalkyl fused to a phenyl ring, C₅₋₇ cycloalkenyl, or, heterocyclyl (itself optionally substituted by oxo, C(O)(C₁₋₆ alkyl), S(O)ₖ(C₁₋₄ alkyl), halo or C₁₋₄ alkyl); k is 0, 1 or 2; or R¹⁰ and R¹¹, and/or R¹⁶ and R¹⁷ may join to form a 4-, 5- or 6-membered ring which optionally includes a nitrogen, oxygen or sulphur atom, said ring being optionally substituted by C₁₋₆ alkyl, S(O)₂(C₁₋₄ alkyl) or C(O)(C₁₋₆ alkyl).

In yet another aspect of the invention R¹¹, R¹², R¹⁴, R¹⁷ and R¹⁹ are C₁₋₈ alkyl (optionally substituted by halo (such as fluoro)), phenyl (optionally substituted as recited above), C₃₋₆ cycloalkyl (optionally substituted by halo (such as fluoro)) or C-linked nitrogen containing heterocyclyl (optionally substituted on the ring nitrogen).

In another aspect of the invention R¹ is NR¹³C(O)R¹⁴, wherein R¹³ and R¹⁴ are as defined above.

In yet another aspect of the invention R¹⁴ is C₁₋₈ alkyl (optionally substituted by halo (such as fluoro, for example to form CF₃CH₂)), phenyl (optionally substituted as recited above), C₃₋₆ cycloalkyl (optionally substituted by halo (such as fluoro, for example to form 1,1-difluorocyclohex-4-yl)) or C-linked nitrogen containing heterocyclyl (such as pyran or piperidine, optionally substituted on the ring nitrogen).

In another aspect the present invention provides a compound of the invention wherein R¹⁴ is C₁₋₈ alkyl (optionally substituted by halo (such as fluoro, for example to form CF₃CH₂)), phenyl (optionally substituted by halo) or C₅₋₆ cycloalkyl (optionally substituted by halo (such as fluoro, for example to form 1,1-difluorocyclohex-4-yl)).

In a further aspect of the invention heterocyclyl is optionally substituted (such as singly substituted for example on a ring nitrogen atom when present) by C₁₋₆ alkyl [optionally substituted by phenyl {which itself optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio or S(O)₂(C₁₋₄ alkyl)} or heteroaryl {which itself optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio or S(O)₂(C₁₋₄ alkyl)}], phenyl {optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio or S(O)₂(C₁₋₄ alkyl)}, heteroaryl {optionally substituted by halo, C₁₋₄ alkyl, C₁₋₄ alkoxy, cyano, nitro, CF₃, (C₁₋₄ akyl)C(O)NH, S(O)₂NH₂, C₁₋₄ alkylthio or S(O)₂(C₁₋₄ alkyl)}, S(O)₂NR⁴⁰R⁴¹, C(O)R⁴², C(O)NHR⁴³ or S(O)₂R⁴⁴; wherein R⁴⁰, R⁴¹, R⁴² and R⁴³ are, independently, hydrogen or C₁₋₆ alkyl; and R⁴⁴ is C₁₋₆ alkyl.

In yet another aspect of the invention R¹ is optionally substituted aryl (such as optionally substituted phenyl) or optionally substituted heteroaryl, wherein the optional substituents are as recited above.

In a further aspect of the invention R¹ is optionally substituted heterocyclyl, such as optionally substituted: piperidin-1-yl, piperidin-4-yl, piperazin-1-yl, pyrrolidin-1-yl, pyrrolidin-3-yl, azetidin-1-yl or azetidin-3-yl.

In a still further aspect of the invention the heterocyclyl of R¹ is mono-substituted by C₁₋₆ alkyl, C₃₋₇ cycloalkyl, phenyl {optionally substituted by halo (for example fluoro), C₁₋₄ alkyl (for example methyl), C₁₋₄ alkoxy (for example methoxy), CF₃ or OCF₃}, S(O)₂(C₁₋₄ alkyl) (for example S(O)₂CH₃, S(O)₂CH₂CH₃ or S(O)₂CH(CH₃)₂), S(O)₂(C₁₋₄ fluoroalkyl) (for example S(O)₂CF₃ or S(O)₂CH₂CF₃), S(O)₂phenyl {optionally substituted (such as mono-substituted) by halo (for example chloro), cyano, C₁₋₄ alkyl, C₁₋₄ alkoxy, CF₃, OCF₃, S(O)₂(C₁₋₄ alkyl) (for example S(O)₂CH₃ or S(O)₂CH₂CH₂CH₃) or S(O)₂(C₁₋₄ fluoroalkyl) (for example S(O)₂CH₂CF₃)}, benzyl {optionally substituted by halo (for example chloro or fluoro), C₁₋₄ alkyl, C₁₋₄ alkoxy (for example methoxy), CF₃ or OCF₃}, C(O)H, C(O)(C₁₋₄ alkyl), benzoyl {optionally substituted by halo (for example chloro or fluoro), C₁₋₄ alkyl (for example methyl), C₁₋₄ alkoxy, CF₃ or OCF₃}, C(O)₂(C₁₋₄ alkyl), C(O)NH₂, C(O)NH(C₁₋₄ alkyl) or C(O)NHphenyl {optionally substituted by halo (for example fluoro), C₁₋₄ alkyl, C₁₋₄ alkoxy, CF₃ or OCF₃}. Said heterocyclyl can also be mono-substituted by S(O)₂NH₂ and S(O)₂N(C₁₋₄ alkyl)₂. In a still further aspect when said heterocyclyl is a 4-substituted piperidin-1-yl, a 1-substituted piperidin-4-yl, a 4-substituted piperazin-1-yl, a 3-substituted pyrrolidin-1-yl, a 1-substituted pyrrolidin-3-yl, a 3-substituted azetidin-1-yl or a 1-substituted azetidin-3-yl (for example where said substituent is as recited earlier in this paragraph). In another aspect said heterocyclyl is a 1-substituted piperidin-4-yl or a 4-substituted piperazin-1-yl, wherein the substituent is S(O)₂(C₁₋₄ alkyl), S(O)₂(C₁₋₄ haloalkyl), S(O)₂(phenyl), S(O)₂N(C₁₋₄ alkyl)₂ or phenyl.

In yet another aspect of the invention R² is phenyl or heteroaryl, either of which is optionally substituted by halo, C₁₋₄ alkyl, C₁₋₄ alkoxy, S(O)ₙ(C₁₋₄ alkyl), nitro, cyano or CF₃; wherein n is 0, 1 or 2, for example 0 or 2. When R² is heteroaryl it is, for example an optionally substituted thiophenyl.

In a still further aspect R² is optionally substituted (for example unsubstituted or substituted in the 2-, 3-, or 3- and 5- positions) phenyl (such as optionally substituted by halo (such as chloro or fluoro), cyano, methyl, ethyl, methoxy, ethoxy or CF₃), or optionally substituted (for example unsubstituted or mono-substituted) heteroaryl (such as optionally substituted by halo (such as chloro or fluoro), cyano, methyl, ethyl, methoxy, ethoxy or CF₃).

In a further aspect the invention provides a compound of the invention wherein R² is phenyl optionally substituted (for example unsubstituted or substituted in the 3-, or 3- and 5-positions) by halo (such as chloro or fluoro) or CF₃.

In another aspect the invention provides a compound of the invention wherein R² is optionally substituted (for example unsubstituted or substituted in the 2-, 3-, or 3- and 5-positions) phenyl (such as optionally substituted by halo (for example chloro or fluoro)). In yet another aspect the invention provides a compound of the invention wherein R² is phenyl, 3-fluorophenyl, 3-chlorophenyl, 3-trifluoromethylphenyl, 3-chloro-5-fluorophenyl or 3,5-difluorophenyl. In a further aspect the invention provides a compound of the invention wherein R² is phenyl, 3-fluorophenyl, 3-chlorophenyl, 3-trifluoromethylphenyl or 3,5-difluorophenyl.

In yet another aspect of the invention R³ is hydrogen or methyl. In a further aspect of the invention when R³ is C₁₋₄ alkyl (such as methyl) and the carbon to which R³ is attached has the R absolute configuration. In yet another aspect of the invention R³ is hydrogen.

In another aspect of the invention X is NR⁶S(O)₂R⁷.

In a further aspect of the invention X is S(O)₂NR⁴R⁵.

In a still further aspect the present invention provides a compound of the invention wherein R⁴, R⁷ and R⁸ are optionally substituted phenyl (the optional substituents being selected from those recited above), optionally substituted benzyl (the phenyl ring being optionally substituted, the substituents being selected from those recited above) or optionally substituted heteroaryl (such as pyridyl, imidazolyl or 1,3,4-thiadiazolyl) (the optional substituents being selected from those recited above).

In yet another aspect the present invention provides a compound of the invention wherein R⁴, R⁷ and R⁸ are phenyl or heteroaryl, each being optionally substituted by OS(O)₂R⁴⁹ or C₁₋₆ alkyl (mono-substituted by S(O)₂R⁵⁰ or C(O)NR⁵¹R⁵²); wherein R⁴⁹, R⁵⁰, R⁵¹ and R⁵² are as defined above, and phenyl may also be substituted by tetrazolyl (itself optionally substituted by C₁₋₄ alkyl).

In a further aspect the present invention provides a compound of the invention wherein R⁴ is phenyl (optionally substituted, for example in the para-position, by halogen (such as chloro or fluoro), cyano, C₁₋₄ alkyl, C₁₋₄ alkoxy, S(C₁₋₄ alkyl), S(O)₂(C₁₋₄ alkyl), OS(O)₂(C₁₋₄ alkyl) or carboxamide), C₃₋₇ cycloalkyl (such as cyclohexyl), pyridyl (optionally substituted by C₁₋₄ alkyl), imidazolyl (optionally substituted by C₁₋₄ alkyl) or 1,3,4-thiadiazolyl (optionally substituted by C₁₋₄ alhyl).

In a still further aspect the invention provides a compound of the invention wherein A is absent.

In another aspect the invention provides a compound of the invention wherein n is 1 or 2.

In yet another aspect the invention provides a compound of the invention wherein n is 1.

In another aspect the invention provides a compound of the invention wherein n is 2.

In a further aspect the invention provides a compound of the invention wherein p is 0.

In a still further aspect the present invention provides a compound of formula (Ia): 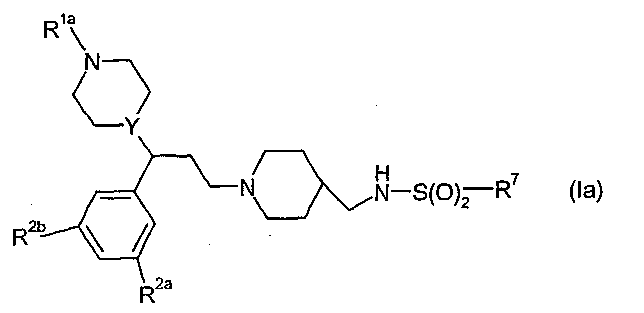 wherein Y is CH or N; R^{1a} is mono-substituted by C₁₋₆ alkyl, C₃₋₇ cycloalkyl, phenyl {optionally substituted by halo (for example fluoro), C₁₋₄ alkyl (for example methyl), C₁₋₄ alkoxy (for example methoxy), CF₃ or OCF₃}, S(O)₂(C₁₋₄ alkyl) (for example S(O)₂CH₃, S(O)₂CH₂CH₃ or S(O)₂CH(CH₃)₂), S(O)₂(C₁₋₄ fluoroalkyl) (for example S(O)₂CF₃ or S(O)₂CH₂CF₃), S(O)₂phenyl {optionally substituted (such as mono-substituted) by halo (for example chloro), cyano, C₁₋₄ alkyl, C₁₋₄ alkoxy, CF₃, OCF₃, S(O)₂(C₁₋₄ alkyl) (for example S(O)₂CH₃ or S(O)₂CH₂CH₂CH₃) or S(O)₂(C₁₋₄ fluoroalkyl) (for example S(O)₂CH₂CF₃)}, benzyl {optionally substituted by halo (for example chloro or fluoro), C₁₋₄ alkyl, C₁₋₄ alkoxy (for example methoxy), CF₃ or OCF₃}, C(O)H, C(O)(C₁₋₄ alkyl), benzoyl {optionally substituted by halo (for example chloro or fluoro), C₁₋₄ alkyl (for example methyl), C₁₋₄ alkoxy, CF₃ or OCF₃}, C(O)₂(C₁₋₄ alkyl), C(O)NH₂, C(O)NH(C₁₋₄ alkyl), C(O)NHphenyl {optionally substituted by halo (for example fluoro), C₁₋₄ alkyl, C₁₋₄ alkoxy, CF₃ or OCF₃} or S(O)₂N(C₁₋₄ alkyl)₂; R^{2a} and R^{2b} are, independently, hydrogen or halo (for example fluoro); and R⁷ is phenyl {optionally substituted by halo (for example fluoro and chloro), C₁₋₄ alkyl (for example methyl), C₁₋₄ alkoxy (for example methoxy), S(O)₂(C₁₋₄ alkyl) (for example S(O)₂CH₃, S(O)₂(C₁₋₄ fluoroalkyl) (for example S(O)₂CF₃), NH(CO)(C₁₋₄ alkyl) (for example NH(C(O))Me), NH(S(O)₂)(C₁₋₄ alkyl) (for example NH(S(O)₂)Me), NH(S(O)₂)(C₁₋₄ fluoroalkyl) (for example NH(S(O)₂)CF₃), 4-nitrophenyl or 4-cyanophenyl.

In a further aspect the present invention provides a compound of formula (Ib): 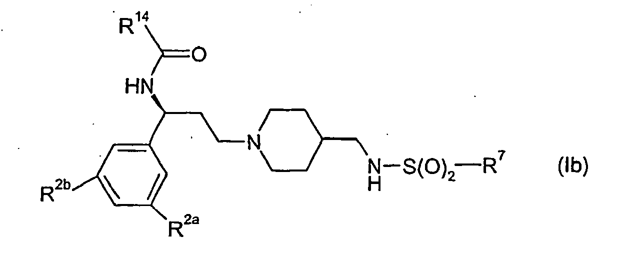 wherein R^{2a}, R^{2b}, R¹⁴ and R⁷ are as defined above.

In a still further aspect the present invention provides a compound of formula (Ic): 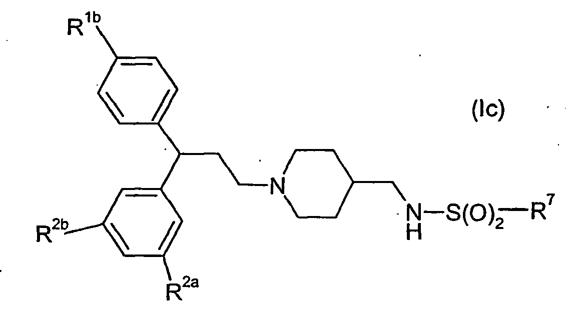 wherein R^{1b} is halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), CO₂H, CO₂(C₁₋₄ alkyl), NHC(O)(C₁₋₄ alkyl), NHS(O)₂(C₁₋₄ alkyl), CF₃, CHF₂, CH₂F, CH₂CF₃ or OCF₃; and R^{2a}, R^{2b} and R⁷ are as defined above.

In another aspect the present invention provides a compound of formula (Id): 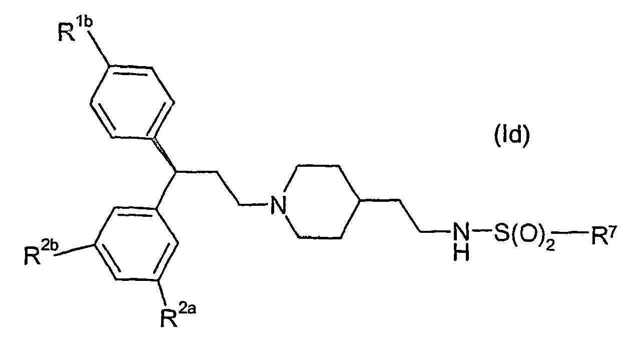 wherein R^{1b}, R^{2a}, R^{2b} and R⁷ are as defined above.

In a further aspect the present invention provides a compound of formula (Ie): 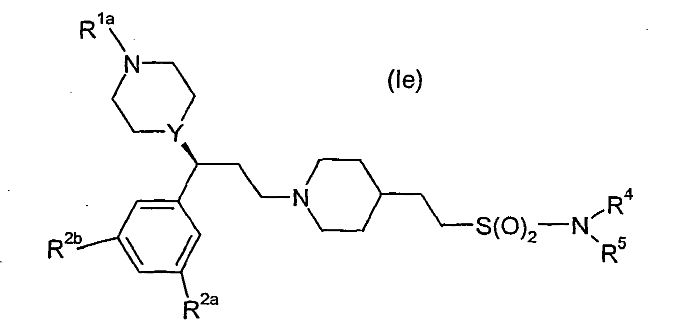 wherein R^{1a}, R^{2a}, R^{2b}, R⁴ and R⁵ are as defined above.

In a still further aspect the present invention provides a compound of formula (If) 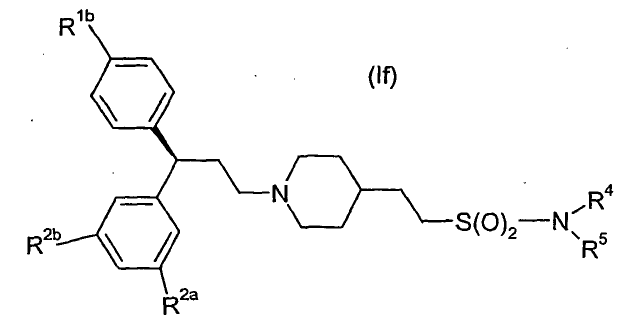 wherein R^{1b}, R^{2a}, R^{2b}, R⁴ and R⁵ are as defined above.

In yet another aspect of the invention there is provided a compound of formula (Ia), (Ib), (Ic) (Id), (Ie) or (If) [such as a compound of formula (Ia), (Ib), (Ic) or (Id) {for example a compound of formula (Ia), (Ib) or (Ic)}] wherein R^{2a} and R^{2b} are, independently, hydrogen, halo (such as chloro or fluoro) or CF₃.

In a still further aspect of the invention there is provided a compound of formula (I), (Ia.), (Ib) or (Ic) wherein R⁷ is phenyl substituted by S(O)₂(C₁₋₄ alkyl) (for example CH₃S(O)₂), CN, NH₂, (C₁₋₄ alkyl)S(O)₂NH (for example CH₃S(O)₂NH), (C₁₋₄ alkyl)C(O)NH (for example CH₃C(O)NH), C₁₋₄ alkoxy (for example CH₃O), (C₁₋₄ alkyl)S(O)₂O (for example CH₃S(O)₂O) or tetrazolyl substituted by C₁₋₄ alkyl (for example 1-methyltetrazol-5-yl or 2-methyltetrazol-5-yl).

In another aspect of the invention there is provided a compound of formula (I), (Ia), (Ib) or (Ic) wherein R⁷ is C₁₋₄ alkyl (such as methyl), di(C₁₋₄ alkyl)amino (such as dimethylamino) or phenyl (the phenyl being optionally substituted by: halogen (such as chloro or fluoro), nitro, cyano, C₁₋₄ alkyl (such as methyl), S(O)₂(C₁₋₄ alkyl) (for example CH₃S(O)₂), (C₁₋₄ alkyl)S(O)₂NH (for example CH₃S(O)₂NH) or (C₁₋₄ haloalkyl)S(O)₂NH (for example CF₃S(O)₂NH)).

In a further aspect of the invention there is provided a compound of formula (I), (Ia), (Ib), (Ic) or (Id) wherein R⁷ is C₁₋₄ alkyl (such as methyl), heteroaryl (such as thienyl or pyridyl) optionally substituted by heteroaryl (such as pyridyl), di(C₁₋₄ alkyl)amino (such as dimethylamino) or phenyl {the phenyl being optionally substituted by: halogen (such as chloro or fluoro), nitro, cyano, amino, phenoxy, C₁₋₄ alkyl (such as methyl), CF₃, C₁₋₄ alkoxy (such as methoxy), S(O)₂(C₁₋₄ alkyl) (for example CH₃S(O)₂), (C₁₋₄ alkyl)S(O)₂NH (for example CH₃S(O)₂NH), (C₁₋₄ haloalkyl)S(O)₂NH (for example CF₃S(O)₂NH), tetrazolyl or (C₁₋₄ alkyl)C(O)NH (for example CH₃C(O)NH or (CH₃)₂CHC(O)NH)}

In yet another aspect of the invention there is provided a compound of formula (I), (Ie) or (If) wherein R⁴ is C₁₋₄ alkyl (optionally substituted by hydroxy or C₁₋₄ alkoxy) or C₃₋₆ cycloalkyl; and R⁵ is hydrogen or C₁₋₄ alkyl.

In a further aspect of the invention there is provided a compound of formula (Ia) or (Ie) wherein R^{1a} is S(O)₂(C₁₋₄ alkyl).

In a still further aspect of the invention there is provided a compound of formula (Ic), (Id), or (If) wherein R^{1b} is S(O)₂(C₁₋₄ alkyl).

In another aspect the present invention provides a compound of formula (I) wherein n is 1 or 2; A is absent; R¹ is phenyl {optionally substituted by S(O)₂(C₁₋₄ alkyl) (for example CH₃S(O)₂)} or piperidin-4-yl {optionally substituted, for example on the 1-position, by S(O)₂(C₁₋₄ alkyl) (for example CH₃S(O)₂)}; R² is phenyl optionally substituted by halogen (for example fluoro, such as 3-fluoro or 3,5-difluoro); R³ is hydrogen; X is NHS(O)₂R⁷; and R⁷ is C₁₋₄ alkyl (for example methyl), di(C₁₋₄ alkyl)amino (for example dimethylamino), phenyl {optionally substituted by: halogen (such as chloro or fluoro), nitro, cyano, amino, C₁₋₄ alkyl (such as methyl), C₁₋₄ haloalkyl (for example CF₃), C₁₋₄ alkoxy (such as methoxy), phenoxy, S(O)₂(C₁₋₄ alkyl) (for example CH₃S(O)₂), (C₁₋₄ alkyl)S(O)₂NH (for example CH₃S(O)₂NH, (CH₃)₂CHS(O)₂NH or (CH₃)₃CS(O)₂NH), (C₁₋₄ haloalkyl)S(O)₂NH (for example CF₃S(O)₂NH), (C₁₋₄ alkyl)C(O)NH (for example CH₃C(O)NH) or tetrazolyl}, thienyl {optionally substituted by pyridinyl} or pyridinyl.

The compounds listed in Tables I, II, III, IV and V illustrate the invention.

**Table I**

| Table I comprises compounds of formula (Ia) | | | | | | |
|---|---|---|---|---|---|---|
| 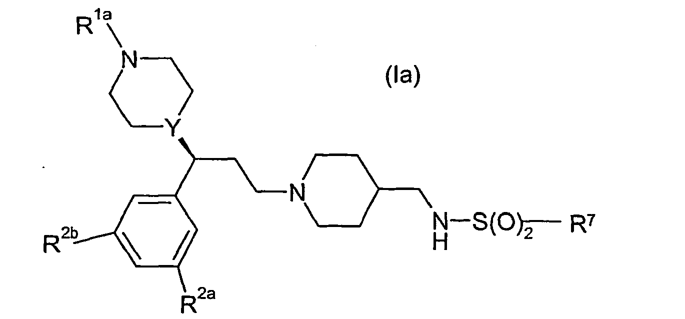 | | | | | | |
| Compound No | R^{1a} | R^{2a} | R^{2b} | Y | R⁷ | MS (MH+) |
| 1 | S(O)₂CH₃ | F | F | CH | 4-S(O)₂CH₃-phenyl | 648 |
| 2 | S(O)₂CH₃ | F | F | CH | 4-nitrophenyl | 615 |
| 3 | S(O)₂CH₃ | F | F | CH | 4-aminophenyl | 585 |
| 4 | S(O)₂CH₃ | F | F | CH | 4-acetamidophenyl | 627 |
| 5 | S(O)₂CH₃ | F | F | CH | 4-methanesulphonylaminophenyl | 663 |
| 6 | S(O)₂CH₃ | F | F | CH | 4-methoxyphenyl | 600 |
| 7 | S(O)₂CH₃ | F | F | CH | 3,4-dimethoxyphenyl | 630 |
| 8 | S(O)₂CH₃ | F | F | CH | 4-phenoxyphenyl | 662 |

**Table II**

| Table II comprises compounds of formula (Ic) | | | | | |
|---|---|---|---|---|---|
| 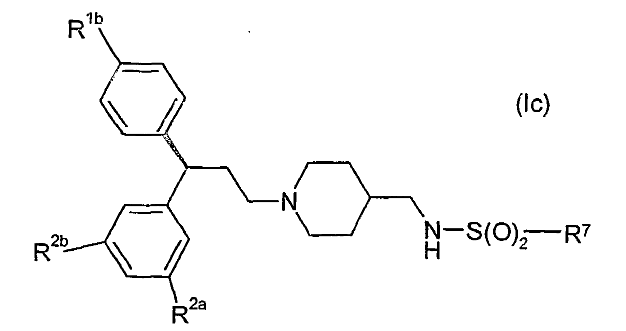 | | | | | |

| Compound No | R^{1b} | R^{2a} | R^{2b} | R⁷ | MS (MH+) |
|---|---|---|---|---|---|
| 1 | S(O)₂CH₃ | F | F | 4-methanesulphonylphenyl | |
| 2 | S(O)₂CH₃ | F | F | methyl | |
| 3 | S(O)₂CH₃ | F | F | dimethylamino | |
| 4 | S(O)₂CH₃ | F | F | 4-methylphenyl | |
| 5 | S(O)₂CH₃ | F | F | 4-nitrophenyl | |
| 6 | S(O)₂CH₃ | F | F | 2-fluorophenyl | |
| 7 | S(O)₂CH₃ | F | F | 3-fluorophenyl | |
| 8 | S(O)₂CH₃ | F | F | 4-fluorophenyl | |
| 9 | S(O)₂CH₃ | F | F | 2,4-difluorophenyl | |
| 10 | S(O)₂CH₃ | F | F | 2-chlorophenyl | |
| 11 | S(O)₂CH₃ | F | F | 3-chlorophenyl | |
| 12 | S(O)₂CH₃ | F | F | 4-chlorophenyl | |
| 13 | S(O)₂CH₃ | F | F | 4-cyanophenyl | |
| 14 | S(O)₂CH₃ | F | F | 4-aminophenyl | |
| 15 | S(O)₂CH₃ | F | F | 4-methanesulphonylaminophenyl | |
| 16 | S(O)₂CH₃ | F | F | 4-trifluoromethylsulphonylaminophenyl | |
| 17 | S(O)₂CH₃ | F | F | 3-cyanophenyl | 588 |
| 18 | S(O)₂CH₃ | F | F | 3-(5-tetrazolyl)phenyl | 631 |
| 19 | S(O)₂CH₃ | F | F | 4-(5-tetrazolyl)phenyl | 631 |
| 20 | S(O)₂CH₃ | F | F | 2-thienyl | 569 |
| 21 | S(O)₂CH₃ | F | F | 3-pyridyl | 564 |
| 22 | S(O)₂CH₃ | F | F | 4-acetamidophenyl | 620 |
| 23 | S(O)₂CH₃ | F | F | 4-amino-3-chlorophenyl | 612 |
| 24 | S(O)₂CH₃ | F | F | 5-(2-pyridyl)-thien-2-yl | 64.6 |
| 25 | S(O)₂CH₃ | F | F | 4-nitro-2-methoxyphenyl | 638 |
| 26 | S(O)₂CH₃ | F | F | 4-amino-2-methoxyphenyl | 608 |
| 27 | S(O)₂CH₃ | F | F | 2-methoxy-4-methanesulphonylphenyl | 686 |
| 28 | S(O)₂CH₃ | F | F | 2-methoxy-4-acetamidophenyl | 652 |
| 29 | S(O)₂CH₃ | F | F | 2-methoxy-4-isopropylamidophenyl | 678 |
| 30 . | S(O)₂CH₃ | F | F | 2-methoxy-4-*tert*-butylamidophenyl | 692 |
| 31 | S(O)₂CH₃ | F | F | 3,4-difluorophenyl | 599 |
| 32 | S(O)₂CH₃ | F | F | 4-amino-3-trifluoromethylphenyl | 646 |
| 33 | S(O)₂CH₃ | F | F | 4-isopropylamido-3-trifluoromethylphenyl | 716 |

**Table III**

| Table III comprises compounds of formula (Id) | | | | | |
|---|---|---|---|---|---|
| 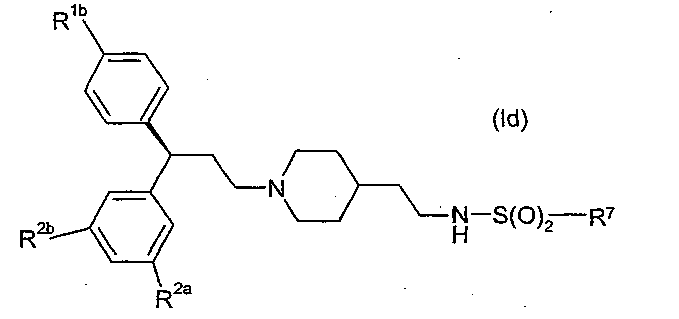 | | | | | |
| Compound No | R^{1b} | R^{2a} | R^{2b} | R⁷ | MS (MH+) |
| 1 | S(O)₂CH₃ | F | F | methyl | 515 |
| 2 | S(O)₂CH₃ | F | F | 4-methanesulphonylphenyl | 655 |
| 3 | S(O)₂CH₃ | F | F | 4-nitrophenyl | 622 |
| 4 | S(O)₂CH₃ | F | F | 4-aminophenyl | 592 |
| 5 | S(O)₂CH₃ | F | F | 4-methanesulphonylaminophenyl | 670 |

**Table IV**

| Table IV comprises compounds of formula (Ie) | | | | | | | |
|---|---|---|---|---|---|---|---|
| 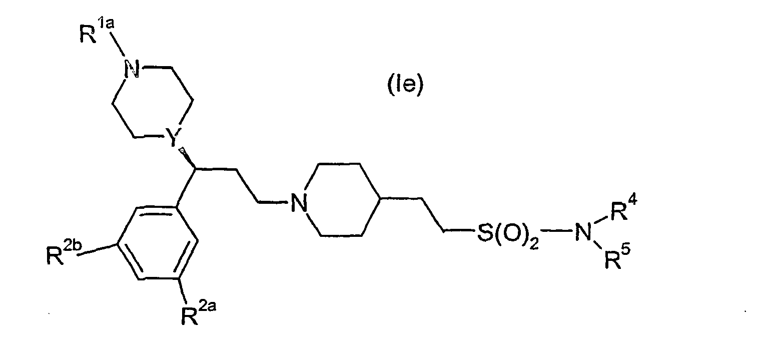 | | | | | | | |
| Compound No | R^{1a} | R^{2a} | R^{2b} | Y | R⁴ | R⁵ | MS (MH+) |
| 1 | S(O)₂CH₃ | F | F | CH | (CH₂)₂OH | H | |
| 2 | S(O)₂CH₃ | F | F | CH | (CH₂)₂OCH₃ | H | 566 |

**Table V**

| Table V comprises compounds of formula (If) | | | | | | |
|---|---|---|---|---|---|---|
| 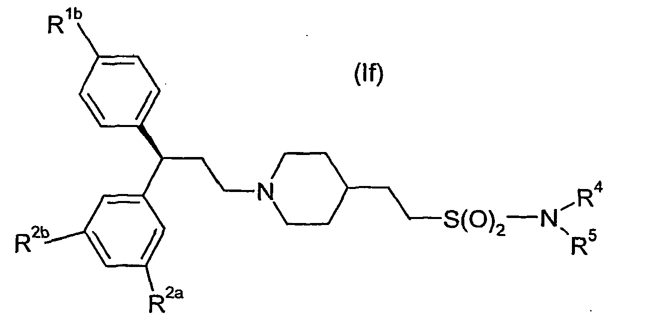 | | | | | | |
| Compound No | R^{1b} | R^{2a} | R^{2b} | R⁴ | R⁵ | MS (MH+) |
| 1 | S(O)₂CH₃ | F | F | (CH₂)₂OH | H | |
| 2 | S(O)₂CH₃ | F | F | cyclopropyl | H | 541 |
| 3 | S(O)₂CH₃ | F | F | CH₃ | CH₃ | 529 |
| 4 | S(O)₂CH₃ | F | F | cyclopentyl | H | 569 |
| 5 | S(O)₂CH₃ | F | F | 2-methoxyethyl | H | 559 |
| 6 | S(O)₂CH₃ | F | F | CH₃CH₂ | CH₃CH₂ | 557 |

In yet another aspect the invention provides each individual compound listed in the tables above.

The compounds of formula (I), (Ia), (Ib), (Ic), (Id), (Ie) and (If) are all compounds of the invention can be prepared as shown below.

A compound of the invention wherein R¹ is an N-linked optionally substituted heterocycle can be prepared by reacting a compound of formula (II): 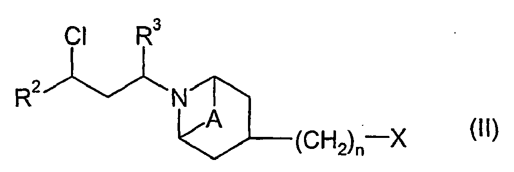 wherein R², R³, n, A and X are as defined above, with a compound R¹H (wherein the H is on a heterocycle ring nitrogen atom) wherein R¹ is as defined above, in the presence of a suitable base (for example a tri(C₁₋₆ alkyl)amine such as triethylamine or Hunig's base), in a suitable solvent (such as a chlorinated solvent, for example dichloromethane) and, for example, at a room temperature (for example 10-30°C), optionally in the presence of sodium iodide.

A compound of the invention, wherein R³ is hydrogen, can be prepared by coupling a compound of formula (III): 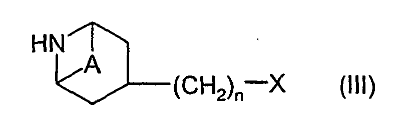 wherein n, A and X are as defined above, with a compound of formula (IV): 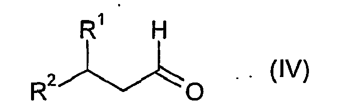 wherein R¹ and R² are as defined above, in the presence of NaBH(OAc)₃ (wherein Ac is C(O)CH₃) in a suitable solvent (such as a chlorinated solvent, for example dichloromethane) at room temperature (for example 10-30°C).

A compound of the invention, wherein R³ is hydrogen, can be prepared by coupling a compound of formula (III): 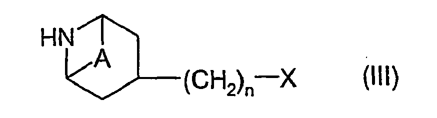 wherein n, A and X are as defined above, with a compound of formula (V): 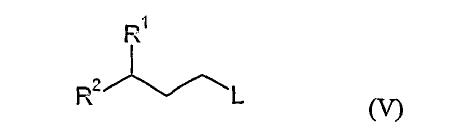 wherein R¹ and R² are as defined above and L is a leaving group such as halogen, tosylate, mesylate or triflate, in the presence of a base, such as potassium carbonate, in a suitable solvent (such as dioxane, acetonitrile or isopropanol) at a temperature from 60°C up to the boiling point of the solvent.

Alternatively, compounds of the invention can be prepared according to Schemes 1-7 (below).

Alternatively, compounds of the invention can be prepared by using or adapting methods described in WO01/87839, EP-A1-1013276, WO00/08013, WO99/38514, WO99/04794, WO00/76511, WO00/76512, WO00/76513, WO00/76514, WO00/76972 or US 2002/0094989.

The starting materials for these processes are either commercially available or can be prepared by literature methods, adapting literature methods or by following or adapting Methods herein described.

In a still further aspect the invention provides processes for preparing the compounds of formula (I), (Ia), (Ib), (Ic), (Id), (Ie) and (If). Many of the intermediates in the processes are novel and these are provided as further features of the invention.

The compounds of the invention have activity as pharmaceuticals, in particular as modulators (such as agonists, partial agonists, inverse agonists or antagonists) of chemokine receptor (such as CCR5) activity, and may be used in the treatment of autoimmune, inflammatory, proliferative or hyperproliferative diseases, or immunologically-mediated diseases (including rejection of transplanted organs or tissues and Acquired Immunodeficiency Syndrome (AIDS)).

The compounds of the present invention are also of value in inhibiting the entry of viruses (such as human immunodeficiency virus (HIV)) into target cells and, therefore, are of value in the prevention of infection by viruses (such as HIV), the treatment of infection by viruses (such as HIV) and the prevention and/or treatment of acquired immune deficiency syndrome (AIDS).

According to a further feature of the invention there is provided a compound of the formula (I), (Ia), (Ib), (Ic), (Id), (Ie) or (If) (such as a compound of formula (I), (Ia), (Ib) or (Ic)), or a pharmaceutically acceptable salt thereof or a solvate thereof, for use in a method of treatment of a warm blooded animal (such as man) by therapy (including prophylaxis).

According to a further feature of the present invention there is provided a method for modulating chemokine receptor activity (such as CCR5 receptor activity) in a warm blooded animal, such as man, in need of such treatment, which comprises administering to said animal an effective amount of a compound of the present invention, or a pharmaceutically acceptable salt thereof or a solvate thereof.

The present invention also provides the use of a compound of the formula (I), (Ia), (Ib), (Ic), (Id), (Ie) or (If) (such as a compound of formula (I), (Ia), (Ib) or (Ic)), or a pharmaceutically acceptable salt thereof or a solvate thereof, as a medicament, such as a medicament for the treatment of transplant rejection, respiratory disease, psoriasis or rheumatoid arthritis (such as rheumatoid arthritis). [Respiratory disease is, for example, COPD, asthma {such as bronchial, allergic, intrinsic, extrinsic or dust asthma, particularly chronic or inveterate asthma (for example late asthma or airways hyper-responsiveness)} or rhinitis {acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofoulous rhinitis; seasonal rhinitis including rhinitis nervosa (hay fever) or vasomotor rhinitis}; and is particularly asthma or rhinitis].

In another aspect the present invention provides the use of a compound of the formula (I), (Ia), (Ib), (Ic), (Id), (Ie) or (If) (such as a compound of formula (I), (Ia), (Ib) or (Ic)), or a pharmaceutically acceptable salt thereof or a solvate thereof, in the manufacture of a medicament for use in therapy (for example modulating chemokine receptor activity (such as CCR5 receptor activity (such as rheumatoid arthritis)) in a warm blooded animal, such as man).

The invention also provides a compound of the formula (I), (Ia), (Ib), (Ic), (Id), (Ie) or (If) (such as a compound of formula (I), (Ia), (Ib) or (Ic)), or a pharmaceutically acceptable salt thereof or a solvate thereof, for use as a medicament, such as a medicament for the treatment of rheumatoid arthritis.

In another aspect the present invention provides the use of a compound of the formula (I), (Ia), (Ib), (Ic), (Id), (Ie) or (If) (such as a compound of formula (I), (Ia), (Ib) or (Ic)), or a pharmaceutically acceptable salt thereof or a solvate thereof, in the manufacture of a medicament for use in therapy (for example modulating chemokine receptor activity (such as CCR5 receptor activity (such as rheumatoid arthritis)) in a warm blooded animal, such as man).

The invention further provides the use of a compound of formula (I), (Ia), (Ib), (Ic), (Id), (Ie) or (If) (such as a compound of formula (I), (Ia), (Ib) or (Ic)), or a pharmaceutically acceptable salt thereof, in the manufacture of a medicament for use in the treatment of:
(1) (the respiratory tract) obstructive diseases of airways including: chronic obstructive pulmonary disease (COPD) (such as irreversible COPD); asthma {such as bronchial, allergic, intrinsic, extrinsic or dust asthma, particularly chronic or inveterate asthma (for example late asthma or airways hyper-responsiveness)}; bronchitis {such as eosinophilic bronchitis}; acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofoulous rhinitis; seasonal rhinitis including rhinitis nervosa (hay fever) or vasomotor rhinitis; sarcoidosis; farmer's lung and related diseases; nasal polyposis; fibroid lung or idiopathic interstitial pneumonia;
(2) (bone and joints) arthrides including rheumatic, infectious, autoimmune, seronegative spondyloarthropathies (such as ankylosing spondylitis, psoriatic arthritis or Reiter's disease), Behçet's disease, Sjogren's syndrome or systemic sclerosis;
(3) (skin and eyes) psoriasis, atopic dermatitis, contact dermatitis or other eczmatous dermitides, seborrhoetic dermatitis, Lichen planus, Phemphigus, bullous Phemphigus, Epidermolysis bullosa, urticaria, angiodermas, vasculitides erythemas, cutaneous eosinophilias, uveitis, Alopecia areata or vernal conjunctivitis;
(4) (gastrointestinal tract) Coeliac disease, proctitis, eosinophilic gastro-enteritis, mastocytosis, Crohn's disease, ulcerative colitis, irritable bowel disease or food-related allergies which have effects remote from the gut (for example migraine, rhinitis or eczema);
(5) (Allograft rejection) acute and chronic following, for example, transplantation of kidney, heart, liver, lung, bone marrow, skin or cornea; or chronic graft versus host disease; and/or
(6) (other tissues or diseases) Alzheimer's disease, multiple sclerosis, atherosclerosis, Acquired Immunodeficiency Syndrome (AIDS), Lupus disorders (such as lupus erythematosus or systemic lupus), erythematosus, Hashimoto's thyroiditis, myasthenia gravis, type I diabetes, nephrotic syndrome, eosinophilia fascitis, hyper IgE syndrome, leprosy (such as lepromatous leprosy), Peridontal disease, Sezary syndrome, idiopathic thrombocytopenia pupura or disorders of the menstrual cycle;
in a warm blooded animal, such as man.

The present invention further provides a method of treating a chemokine mediated disease state (such as a CCR5 mediated disease state) in a warm blooded animal, such as man, which comprises administering to a mammal in need of such treatment an effective amount of a compound of formula (I), (Ia), (Ib), (Ic), (Id), (Ie) or (If) (such as a compound of formula (I), (Ia), (Ib) or (Ic)), or a pharmaceutically acceptable salt thereof or solvate thereof.

In order to use a compound of the invention, or a pharmaceutically acceptable salt thereof or solvate thereof, for the therapeutic treatment of a warm blooded animal, such as man, in particular modulating chemokine receptor (for example CCR5 receptor) activity, said ingredient is normally formulated in accordance with standard pharmaceutical practice as a pharmaceutical composition.

Therefore in another aspect the present invention provides a pharmaceutical composition which comprises a compound of the formula (I), (Ia), (Ib), (Ic), (Id), (Ie) or (If) (such as a compound of formula (I), (Ia), (Ib) or (Ic)), or a pharmaceutically acceptable salt thereof or a solvate thereof (active ingredient), and a pharmaceutically acceptable adjuvant, diluent or carrier. In a further aspect the present invention provides a process for the preparation of said composition which comprises mixing active ingredient with a pharmaceutically acceptable adjuvant, diluent or carrier. Depending on the mode of administration, the pharmaceutical composition will, for example, comprise from 0.05 to 99 %w (per cent by weight), such as from 0.05 to 80 %w, for example from 0.10 to 70 %w, such as from 0.10 to 50 %w, of active ingredient, all percentages by weight being based on total composition.

The pharmaceutical compositions of this invention may be administered in standard manner for the disease condition that it is desired to treat, for example by topical (such as to the lung and/or airways or to the skin), oral, rectal or parenteral administration. For these purposes the compounds of this invention may be formulated by means known in the art into the form of, for example, aerosols, dry powder formulations, tablets, capsules, syrups, powders, granules, aqueous or oily solutions or suspensions, (lipid) emulsions, dispersible powders, suppositories, ointments, creams, drops and sterile injectable aqueous or oily solutions or suspensions.

A suitable pharmaceutical composition of this invention is one suitable for oral administration in unit dosage form, for example a tablet or capsule which contains between 0.1mg and 1g of active ingredient.

In another aspect a pharmaceutical composition of the invention is one suitable for intravenous, subcutaneous or intramuscular injection.

Each patient may receive, for example, an intravenous, subcutaneous or intramuscular dose of 0.01mgkg⁻¹ to 100mgkg⁻¹ of the compound, for example in the range of 0.1mgkg⁻¹ to 20mgkg⁻¹ of this invention, the composition being administered 1 to 4 times per day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose, the composition being administered 1 to 4 times per day.

The following illustrate representative pharmaceutical dosage forms containing the compound of formula (I), (Ia), (Ib), (Ic), (Id), (Ie) or (If) (such as a compound of formula (I), (Ia), (Ib) or (Ic)), or a pharmaceutically acceptable salt thereof or a solvent thereof (hereafter Compound X), for therapeutic or prophylactic use in humans:
(a)

| Tablet I | mg/tablet |
|---|---|
| Compound X | 100 |
| Lactose Ph.Eur. | 179 |
| Croscarmellose sodium | 12.0 |
| Polyvinylpyrrolidone | 6 |
| Magnesium stearate | 3.0 |

(b)

| Tablet II | mg/tablet |
|---|---|
| Compound X | 50 |
| Lactose Ph.Eur. | 229 |
| Croscarmellose sodium | 12.0 |
| Polyvinylpyrrolidone | 6 |
| Magnesium stearate | 3.0 |

(c)

| Tablet III | mg/tablet |
|---|---|
| Compound X | 1.0 |
| Lactose Ph.Eur. | 92 |
| Croscarmellose sodium | 4.0 |
| Polyvinylpyrrolidone | 2.0 |
| Magnesium stearate | 1.0 |

(d)

| Capsule | mg/capsule |
|---|---|
| Compound X | 10 |
| Lactose Ph.Eur. | 389 |
| Croscarmellose sodium | 100 |
| Magnesium stearate | 1.0 |

(e)

| Injection I | (50 mg/ml) |
|---|---|
| Compound X | 5.0% w/v |
| Isotonic aqueous solution | to 100% |

Buffers, pharmaceutically-acceptable cosolvents such as polyethylene glycol, polypropylene glycol, glycerol or ethanol or complexing agents such as hydroxy-propyl β-cyclodextrin may be used to aid formulation.

The above formulations may be obtained by conventional procedures well known in the pharmaceutical art. The tablets (a)-(c) may be enteric coated by conventional means, for example to provide a coating of cellulose acetate phthalate.

The invention further relates to combination therapies or compositions wherein a compound of formula (I), (Ia), (Ib), (Ic), (Id), (Ie) or (If), or a pharmaceutically acceptable salt, solvate or a solvate of a salt thereof, or a pharmaceutical composition comprising a compound of formula (I), (Ia), (Ib), (Ic), (Id), (Ie) or (If), or a pharmaceutically acceptable salt, solvate or a solvate of a salt thereof, is administered concurrently (possibly in the same composition) or sequentially with an agent for the treatment of any one of the above disease states.

In particular, for the treatment of the inflammatory diseases rheumatoid arthritis, psoriasis, inflammatory bowel disease, COPD, asthma and allergic rhinitis a compound of the invention can be combined with a TNF-α inhibitor (such as an anti-TNF monoclonal antibodie (such as Remicade, CDP-870 and D.sub2.E.sub7.), or a TNF receptor immunoglobulin molecule (such as Enbrel.reg.)), a non-selective COX-1 / COX-2 inhibitor (such as piroxicam or diclofenac; a propionic acid such as naproxen, flubiprofen, fenoprofen, ketoprofen or ibuprofen; a fenamate such as mefenamic acid, indomethacin, sulindac or apazone; a pyrazolone such as phenylbutazone; or a salicylate such as aspirin), a COX-2 inhibitor (such as meloxicam, celecoxib, rofecoxib, valdecoxib or etoricoxib) low dose methotrexate, lefunomide; ciclesonide; hydroxychloroquine, d-penicillamine or auranofin, or parenteral or oral gold.

The present invention still further relates to the combination of a compound of the invention together with:
- a leukotriene biosynthesis inhibitor, a 5-lipoxygenase (5-LO) inhibitor or a 5-lipoxygenase activating protein (FLAP) antagonist, such as zileuton, ABT-761, fenleuton, tepoxalin, Abbott-79175, Abbott-85761, an N-(5-substituted)-thiophene-2-alkylsulfonamide, a 2,6-di-tert-butylphenol hydrazones, a methoxytetrahydropyran such as Zeneca ZD-2138, SB-210661, a pyridinyl-substituted 2-cyanonaphthalene compound such as L-739,010; a 2-cyanoquinoline compound such as L-746,530; an indole or quinoline compound such as MK-591, MK-886 or BAY x 1005;
- a receptor antagonist for a leukotriene LTB.sub4., LTC.sub4., LTD.sub4. or LTE.sub4. selected from the group consisting of a phenothiazin-3-one such as L-651,392; an amidino compound such as CGS-25019c; a benzoxalamine such as ontazolast; a benzenecarboximidamide such as BIIL, 284/260; or a compound such as zafirlukast, ablukast, montelukast, pranlukast, verlukast (MK-679), RG-12525, Ro-245913, iralukast (CGP 45715A) or BAY x 7195;
- a PDE4 inhibitor including an inhibitor of the isoform PDE4D;
- an antihistaminic H.sub1. receptor antagonist such as cetirizine, loratadine, desloratadine, fexofenadine, astemizole, azelastine or chlorpheniramine;
- a gastroprotective H.sub2. receptor antagonist;
- an α.sub1.-and α.sub2.-adrenoceptor agonist vasoconstrictor sympathomimetic agent, such as propylhexedrine, phenylephrine, phenylpropanolamine, pseudoephedrine, naphazoline hydrochloride, oxymetazoline hydrochloride, tetrahydrozoline hydrochloride, xylometazoline hydrochloride or ethylnorepinephrine hydrochloride;
- an anticholinergic agent such as ipratropium bromide, tiotropium bromide, oxitropium bromide, pirenzepine or telenzepine;
- a β.sub1.- to β.sub4.-adrenoceptor agonist such as metaproterenol, isoproterenol, isoprenaline, albuterol, salbutamol, formoterol, salmeterol, terbutaline, orciprenaline, bitolterol mesylate or pirbuterol, or a methylxanthanine including theophylline and aminophylline; sodium cromoglycate; or a muscarinic receptor (M1, M2, and M3) antagonist;
- an insulin-like growth factor type I (IGF-1) mimetic;
- an inhaled glucocorticoid with reduced systemic side effects, such as prednisone, prednisolone, flunisolide, triamcinolone acetonide, beclomethasone dipropionate, budesonide, fluticasone propionate or mometasone furoate;
- an inhibitor of a matrix metalloprotease (MMP), such as a stromelysin, a collagenase, or a gelatinase or aggrecanase; such as collagenase-1 (MMP-1), collagenase-2 (MMP-8), collagenase-3 (MMP-13), stromelysin-1 (MMP-3), stromelysin-2 (MMP-10), and stromelysin-3 (MMP-11) or MMP-12 ;
- a modulator of ohemokine receptor function such as CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family;
- an osteoporosis agent such as roloxifene, droloxifene, lasofoxifene or fosomax;
- an immunosuppressant agent such as FK-506, rapamycin, cyclosporine, azathioprine or methotrexate; or,
- an existing therapeutic agent for the treatment of osteoarthritis, for example a non-steroidal anti-inflammatory agent (hereinafter NSAID's) such as piroxicam or diclofenac, a propionic acid such as naproxen, flubiprofen, fenoprofen, ketoprofen or ibuprofen, a fenamate such as mefenamic acid, indomethacin, sulindac or apazone, a pyrazolone such as phenylbutazone, a salicylate such as aspirin, a COX-2 inhibitor such as celecoxib, valdecoxib, rofecoxib or etoricoxib, an analgesic or intra-articular therapy such as a corticosteroid or a hyaluronic acid such as hyalgan or synvisc, or a P2X7 receptor antagonist.

The present invention still further relates to the combination of a compound of the invention together with: (i) a tryptase inhibitor; (ii) a platelet activating factor (PAF) antagonist; (iii) an interleukin converting enzyme (ICE) inhibitor; (iv) an IMPDH inhibitor; (v) an adhesion molecule inhibitor including a VLA-4 antagonist; (vi) a cathepsin; (vii) a MAP kinase inhibitor; (viii) a glucose-6 phosphate dehydrogenase inhibitor; (ix) a kinin-B.subl, - and B.sub2. -receptor antagonist; (x) an anti-gout agent, e.g., colchicine; (xi) a xanthine oxidase inhibitor, e.g., allopurinol; (xii) an uricosuric agent, e.g., probenecid, sulfinpyrazone or benzbromarone; (xiii) a growth hormone secretagogue; (xiv) a transforming growth factor (TGFβ); (xv) a platelet-derived growth factor (PDGF); (xvi) a fibroblast growth factor, e.g., basic fibroblast growth factor (bFGF); (xvii) a granulocyte macrophage colony stimulating factor (GM-CSF); (xviii) a capsaicin cream; (xix) a Tachykinin NK.sub1. and NK.sub3. receptor antagonist selected from the group consisting of NKP-608C; SB-233412 (talnetant); and D-4418; (xx) an elastase inhibitors selected from the group consisting of UT-77 and ZD-0892; (xxi) a TNFα converting enzyme inhibitor (TACE); (xxii) an induced nitric oxide synthase inhibitor (iNOS); or (xxiii) a chemoattractant receptor-homologous molecule expressed on TH2 cells (a CRTH2 antagonist).

The invention will now be illustrated by the following non-limiting Examples in which, unless stated otherwise: ,
(i) temperatures are given in degrees Celsius (°C); operations were carried out at room or ambient temperature, that is, at a temperature in the range of 18-25°C;
(ii) organic solutions were dried over anhydrous magnesium sulfate; evaporation of solvent was carried out using a rotary evaporator under reduced pressure (600-4000 Pascals; 4.5-30 mm Hg) with a bath temperature of up to 60°C;
(iii) chromatography unless otherwise stated means flash chromatography on silica gel; thin layer chromatography (TLC) was carried out on silica gel plates; where a "Bond Elut" column is referred to, this means a column containing 10g or 20g of silica of 40 micron particle size, the silica being contained in a 60ml disposable syringe and supported by a porous disc, obtained from Varian, Harbor City, California, USA under the name "Mega Bond Elut SI". Where an "Isolute^{™} SCX column" is referred to, this means a column containing benzenesulphonic acid (non-endcapped) obtained from International Sorbent Technology Ltd., 1st House, Duffryn Industial Estate, Ystrad Mynach, Hengoed, Mid Glamorgan, UK. Where "Argonaut^{™} PS-*tris*-amine scavenger resin" is referred to, this means a *tris-*(2-aminoethyl)amine polystyrene resin obtained from Argonaut Technologies Inc., 887 Industrial Road, Suite G, San Carlos, California, USA.
(iv) in general, the course of reactions was followed by TLC and reaction times are given for illustration only;
(v) yields, when given, are for illustration only and are not necessarily those which can be obtained by diligent process development; preparations were repeated if more material was required;
(vi) when given, ¹H NMR data is quoted and is in the form of delta values for major diagnostic protons, given in parts per million (ppm) relative to tetramethylsilane (TMS) as an internal standard, determined at 300 MHz using perdeuterio DMSO (CD₃SOCD₃) as the solvent unless otherwise stated; coupling constants (J) are given in Hz;
(vii) chemical symbols have their usual meanings; SI units and symbols are used;
(viii) solvent ratios are given in percentage by volume;
(ix) mass spectra (MS) were run with an electron energy of 70 electron volts in the chemical ionisation (APCI) mode using a direct exposure probe; where indicated ionisation was effected by electrospray (ES); where values for m/z are given, generally only ions which indicate the parent mass are reported, and unless otherwise stated the mass ion quoted is the positive mass ion - (M+H)⁺;
(x) LCMS characterisation was performed using a pair of Gilson 306 pumps with Gilson 233 XL sampler and Waters ZMD4000 mass spectrometer. The LC comprised water symmetry 4.6x50 column C18 with 5 micron particle size. The eluents were: A, water with 0.05% formic acid and B, acetonitrile with 0.05% formic acid. The eluent gradient went from 95% A to 95% B in 6 minutes. Where indicated ionisation was effected by electrospray (ES); where values for m/z are given, generally only ions which indicate the parent mass are reported, and unless otherwise stated the mass ion quoted is the positive mass ion - (M+H)⁺;
(xi) PS-NCO resin is an isocyanate resin and is available from Argonaut; and,
(xii) the following abbreviations are used:

| | |
|---|---|
| THF | tetrahydrofuran |
| DMSO | dimethylsulphoxide |
| Boc | tert-butoxycarbonyl |
| DMF | N,N-dhnethylforrnamide |
| DCM | dichloromethane |
| DIPEA | N,N-Diisopropylethylamine |
| R-BINAP | R 2,2'-Bis(diphenylphosphino)-1,1'-binaphthyl |

### Example 1

This Example illustrates the preparation of *N*-[(1-{(3*R*)-3-(3,5-difluorophenyl)-3-[4-(methylsulfonyl)phenyl]propyl}piperidin-4-yl)methyl]-4-(methylsulfonyl)benzenesulfonamide (Compound 1, Table III)

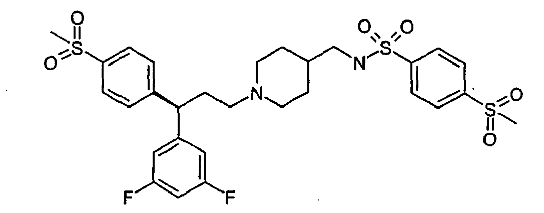

4-Methanesulphonylbenzenesulphonyl chloride (135 mg) was added to a solution of *N*-[(1-{(3*R*)-3-(3,5-difluorophenyl)-3-[4-(methylsulphonyl)phenyl]propyl}piperidin-4-yl)methylamine dihydrochloride (250 mg) and triethylamine (162 mg) in dichloromethane (10 ml) and the mixture was stirred at 20°C for 16 hours. The reaction mixture was washed with water (2x 10 ml), dried and evaporated to dryness. The residue was passed down a Bond Elut column eluting with a solvent gradient (ethyl acetate-30%methanol/ethyl acetate) to give the title compound, yield 176 mg, MH⁺ 641. NMR (DMSOd6): 1.02 (q, 2H), 1.29 (m, 1H), 1.55 (d, 2H), 1.73 (t, 2H), 2.03-2.27 (m, 3H), 2.6-2.78 (m, 3H), 3.27 (s, 6H), 4.17 (t, 1H), 7.0 (m, 1H), 7.10 (d, 2H), 7.6 (d, 2H), 7.80 (m, 3H), 8.0 (d,2H), 8.13 (d, 2H).

### [(1-{(3R)-3-(3,5-difluorophenyl)-3-[4-(methylsulphonyl)phenyl]propyl}piperidin-4-yl)methyl]amine dihydrochloride

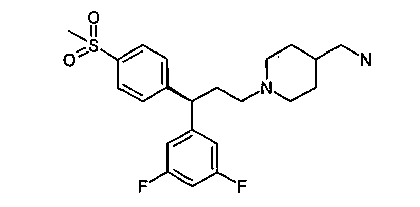

*tert*-Butyl [(1-{(3*R*)-3-(3,5-difluorophenyl)-3-[4-(methylsulphonyl)phenyl]propyl}piperidin-4-yl)methyl]carbamate (1.6g) was dissolved in 4M HCI/dioxane (20 ml) and allowed to stand at room temperature for 2 hours. Diethyl ether (100 ml) was added and the solid obtained was filtered and dried, yield 1.4g. NMR (DMSOd6): 1.58 (m, 1H), 1.70-2.12 (m, 2H), 2.56-2.75 (m, 4H), 2.76-2.96 (m, 4H), 3.17 (s, 3H), 3.5 (d), 3.85 (bs, 6H), 4.28 (t, 1H), 7.06 (t, 1H), 7.19 (d, 2H), 7.65 (d, 2H), 7.86 (d, 2H), 8.08 (bs, 2H).

### tert-Butyl [(1-{(3R)-3-(3,5-difluorophenyl)-3-[4-(methylsulphonyl)phenyl]propyl}piperidin-4-yl)methyl]carbamate

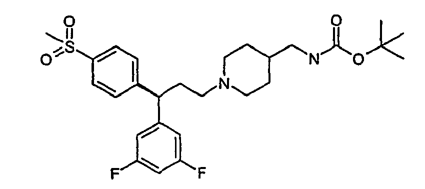

*tert*-Butyl (piperidin-4-ylmethyl)carbamate (680 mg) [commercially available] was added to a solution of (3R)-3-(3,5-difluorophenyl)-3-(4-methanesulphonylphenyl)-propionaldehdye (1g in 30 ml of dichloromethane prepared according to Method C) and sodium triacetoxyborohydride (671 mg) in dichloromethane (20 ml) and the mixture was stirred at room temperature for 3 hours then washed with 2M NaOH (2x50ml), dried and evaporated to dryness. The residue was passed down a Bond Elut column eluting with a solvent gradient (ethyl acetate-20% methanol/ethyl acetate) to give the title compound, yield 1.6g, MH⁺ 523. NMR (DMSOd6): 1.35 (s, 9H), 1.52 (d, 2H), 1.73 (d, 2H), 2.05-2.25 (m, 4H), 2.68-2.80 (m, 4H), 3.27 (s, 1H), 4.17 (t, 1H), 6.74 (m, 1H), 7.0 (t, 1H), 7.11 (d, 2H), 7.62 (d,2H), 7.81 (d, 2H).

### Example 2

This Example illustrates the preparation of N [2-(1-{(3R)-3-(3,5-difluorophenyl)-3-[4-(methylsulfonyl)phenyl]propyl}piperidin-4-yl)ethyl]-4-nitrobenzenesulfonamide (Compound 3 of Table III].

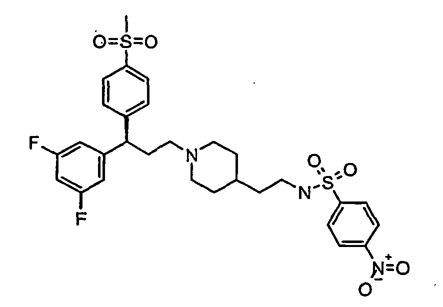

4-Nitrobenzenesulphonyl chloride (0.6 g) was added to a solution of [2-(1-{(3*R*)-3-(3,5-difluorophenyl)-3-[4-(methylsulfonyl)phenyl]propyl}piperidin-4-yl)ethyl]amine (1.2g) (Method F) and pyridine (210 mg) in dichloromethane (50 ml) and the mixture was stirred for 2 hours, washed with water (2x25 ml) and dried. The residue obtained on removal of the solvent was purified by chromatography on silica eluting with a solvent gradient of ethyl acetate: 10% methanol/ethyl acetate to give the title compound, yield 1.6g, MH⁺ 622, NMR (CDCl₃): 1.1-1.9 (m, 13H) 2.8 (m, 2H) 3.03 (m, 5H) 4.1 (m, 1H) 4.6 (Br, 1H) 6.6-6.8 (m, 3H) 7.4 (d, 2H) 7.9 (d, 2H) 8.05 (d, 2H) 8.35 (d, 2H).

Following this method but using [(1-{(*3R*)-3-(3,5-difluorophenyl)-3-[4-(methylsulfonyl)phenyl]propyl}piperidin-4-yl)methyl]amine as starting material there is obtained *N*-[(1-{(3*R*)-3-(3,5-difluorophenyl)-3-[4-(methylsulfonyl)phenyl]propyl}piperidin-4-yl)methyl]-4-nitrobenzenesulfonamide (Compound 5 Table II), MH⁺ 608, NMR (CDCl₃): 1.1-1.9 (m, 11H) 2.8 (m, 2H) 2.9 (m, 2H) 3.05 (s, 3H) 4.1(t, 1H) 4.7 (m, 1H) 6.6-6.8 (m, 3H) 7.4 (d, 2H) 7.8 (d, 2H) 8.05 (d, 2H) 8.4 (d, 2H).

### Example 3

This Example illustrates the preparation of 4-amino-*N*-[2-(1-{(3*R*)-3-(3,5-difluorophenyl)-3-[4-(methylsulfonyl)phenyl]propyl}piperidin-4-yl)ethyl]benzenesulfonamide (Compound 4 in Table III) 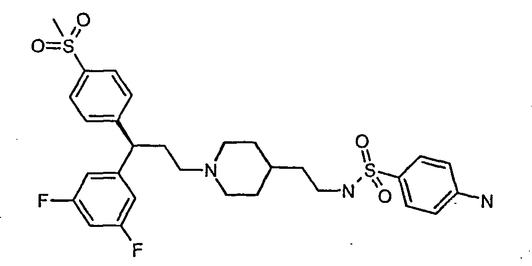 A solution of *N*-[2-(1-{(3*R*)-3-(3,5-difluorophenyl)-3-[4-(methylsulfonyl)phenyl]propyl}piperidin-4-yl)ethyl]-4-nitrobenzenesulfonamide (1.4 g; Example 2) in a mixture of ethanol (100 ml) and THF (50 ml) containing 10% Pd/C (200 mg) as catalyst was hydrogenated at atmospheric pressure. The catalyst was filtered and the filtrate evaporated to dryness to give the title compound, yield 1.01 g, MH⁺ 592, NMR (CDCl₃): 1.05- 1.95 (m, 13H) 2.8 (m, 2H) 2.9 (q, 2H) 3.05 (s, 3H) 4.1 (m, 3H) 4.25 (m, 1H) 6.6-6.8 (m 5H) 7.4 (d, 2H) 7.6 (d, 2H) 7.9 (d, 2H).

Following this method but using *N*-[(1-{(3*R*)-3-(3,5-difluorophenyl)-3-[4-(methylsulfonyl)phenyl]propyl}piperidin-4-yl)methyl]-4-nitrobenzenesulfonamide as starting material there is obtained 4-amino-*N*-[(1-{(3*R*)-3-(3,5-difluorophenyl)-3-[4-(methylsulfonyl)phenyl]propyl}piperidin-4-yl)methyl]benenesulfonamide (Compound 14 in Table II) MH⁺ 578.

### Example 4

This Example illustrates the preparation of *N*-[2-(1-{(3*R*)-3-(3,5-difluorophenyl)-3-[4-(methylsulfonyl)phenyl]propyl}piperidin-4-yl)ethyl]-4-[(methylsulfonyl)amino]-benzenesulfonamide (Compound 5 in Table III).

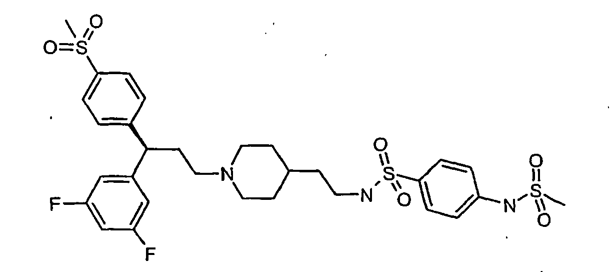

Methanesulphonyl chloride (77 mg) was added to a solution of 4-amino-*N*-[2-(1-{(3*R*)-3-(3,5-difluorophenyl)-3-[4-(methylsulfonyl)phenyl]propyl}piperidin-4-yl)ethyl]benzenesulfonamide (0.4 g; Example 3) and triethylamine (53 mg) in dichloromethane (30 ml) and the mixture was stirred for 16 hours by which time an oil had precipitated out. Saturated aqueous sodium bicarbonate (100 ml) was added and the mixture was stirred for 20 minutes. The aqueous/dichloromethane mixture was decanted and the residue was stirred with water (25 ml) which was decanted in turn. The residue was dissolved in methanol (10 ml) and poured on to a 20g SCX2 cartridge which was eluted with methanol (5x20 ml) and 1M ammonia in methanol (5x20 ml). The methanolic ammonia washings were evaporated to dryness and the resulting glass was triturated with diethyl ether to give the title compound as a solid, yield 176 mg, MH⁺ 670, NMR (CDCl₃): 1.1-1.9 (m, 13H) 2.75 (m, 2H) 2.95 (m, 2H) 3.05 (s, 3H) 3.1(s, 3H) 4.1 (t, 1H) 4.6 (m, 1H) 6.6-6.8 (m, 3H) 7.3-7.5 (m, 4H) 7.8-7.9 (m, 4H).

Following this method but using 4-amino-*N*-[(1-{(3*R*)-3-(3,5-difluorophenyl)-3-[4-(methylsulfonyl)phenyl]propyl}piperidin-4-yl)methyl]benzenesulfonamide as starting material there is obtained *N*-[(1-{(3*R*)-3-(3,5-difluorophenyl)-3-[4-(methylsulfonyl)phenyl]propyl}piperidin-4-yl)methyl]-4-[(methylsulfonyl)amino]benzenesulfonamide (Compound 15 in Table II). MH⁺ 656, NMR (DMSOd₆): 1.3-1.9 (m, 11H) 2.6 (m, 2H) 2.8 (m, 2H) 3.1 (s, 3H) 3.15 (s, 3H) 4.2 (t, 1H) 7-7.2 (m, 3H) 7.3 (d, 2H) 7.6-7.9 (m, 6H)

### Example 5

This Example illustrates the preparation of *N-*[(1-{(3R)-3-(3,5-difluorophenyl)-3-[4-(methylsulfonyl)phenyl]propyl} piperidin-4-yl)methyl]-4- ((trifluoromethyl)sulfonyl]amino}benzenesulfonamide (Compound 16 in Table II).

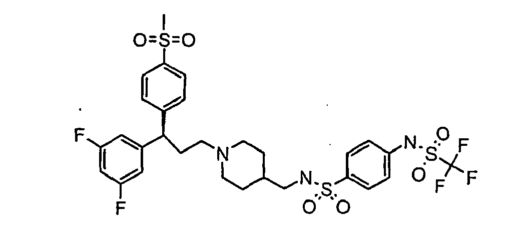

Trifluoromethanesulphonic anhydride (97 mg) was added to a solution of 4-amino-*N-*[(1-{(3*R*)-3-(3,5-difluorophenyl)-3-[4-(methylsulfonyl)phenyl]propyl}piperidin-4-yl)methyl]benzenesulfonamide (200 mg) in pyridine (5 ml) and the mixture was stirred for 2 hours. A further aliquot oftrifluoromethanesulphonic anhydride was added and stirring continued for 16 hours. The solvent was evaporated and the oil obtained was stirred with water (25 ml). The water was decanted off and the oil dissolved in methanol (20 ml) and poured on to a 20g SCX2 cartridge and was eluted with methanol (6x20 ml) and 1M ammonia in methanol (8x20 ml). The methanolic ammonia washings were evaporated to dryness and the residue was stirred with dichloromethane (20 ml) for 20 minutes. The solid product was filtered and dried under vacuum, yield 40 mg MH⁺ 7.10, NMR (DMSOd₆): 1.3-1.9 (m, 11H) 2.6 (m, 2H) 2.8 (m, 2H) 3.15 (s, 3H) (4.2 (t, 1H) 7-7.2 (m, 5H) 7.3 (t, 1H) 7.4 (d, 2H) 7.6 (d, 2H) 7.9 (m, 2H)

### Example 6

This Example illustrates the preparation of *N*-[(1-{(3*R*)-3-(3,5-difluorophenyl)-3-[1-(methylsulfonyl)piperidin-4-yl]propyl}piperidin-4-yl)methyl]-4-nitrobenzenesulfonamide (Compound 2 in Table I).

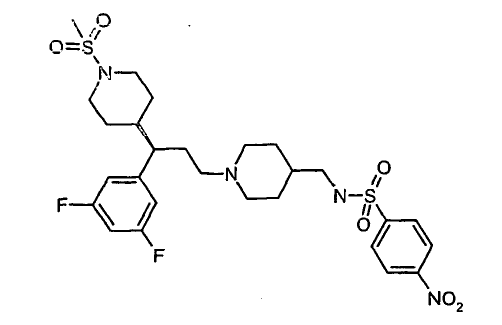

4-Nitrobenzenesulphonyl chloride (0.61 g) was added to a stirred solution of [(1-{(3*R*)-3-(3,5-difluorophenyl)-3-[1-(methylsulfonyl)piperidin-4-yl]propyl }piperidin-4-yl)methyl]amine (1.2 g; Method G) and triethylamine (282 mg) in dichloromethane (75 ml) and stirring was continued for 4 hours. The reaction mixture was washed with water (2x25 ml), dried and evaporated to dryness. The residue obtained on removal of the solvent was purified by chromatography on silica eluting with a-solvent gradient of ethyl acetate: 20% methanol/ethyl acetate and gave the title compound, yield 1.2g, MH⁺ 615, NMR (CDCl₃): 1.1-2.7 (m, 21H) 2.75 (s, 3H) 2.9 (t, 2H) 3.7 (m, 1H) 3.85 (m, 1H) 4.8 (m, 1H) 6.6 (m, 3H) 8.0 (d, 2H) 8.4 (d, 2H).

### Example 7

This Example illustrates the preparation of 4-amino-*N*-[(1-{(3*R*)-3-(3,5-difluorophenyl)-3-[1-(methylsulfonyl)piperidin-4-yl]propyl}piperidin-4-yl)methyl]benzenesulfonamide (Compound 3 in Table I).

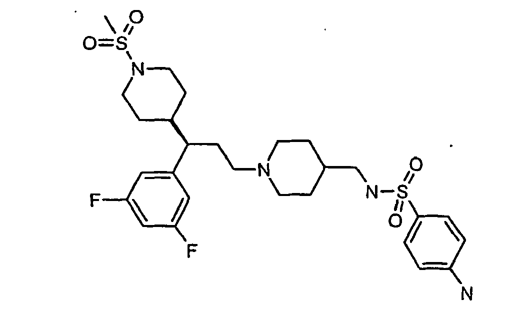

A solution of *N*-[(1-{(3*R*)-3-(3,5-difluorophenyl)-3-[1-(methylsulfonyl)piperidin-4-yl]propyl}piperidin-4-yl)methyl]-4-nitrobenzenesulfonamide (1 g) (Example 5) in THF (100 ml) containing 20% Pd(OH)₂/C as catalyst was hydrogenated under a hydrogen filled balloon. The catalyst was filtered and the filtrate was poured on to a 20g SCX2 cartridge and eluted with methanol (6x20 ml) and 1M ammonia in methanol (6x20 ml). The combined methanolic ammonia washings were evaporated to dryness to give the title compound, yield 1g, MH⁺ 584, NMR (CDCl₃): 1.1-2.7(m, 21H) 2.75 (s, 3H) 2.8 (m, 2H) 3.7 (m, 1H) 3.9 (m, 1H) 4.1 (s, 2H) 4.4 (t, 1H) 6.6 (m, 5H) 7.6 (d, 2H)

### Example 8

This Example illustrates the preparation of *N*-[4-({[(1-{(3*R*)-3-(3,5-difluorophenyl)-3-[1-(methylsulfonyl)piperidin-4-yl]propyl}piperidin-4-yl)methyl]amino}sulfonyl)phenyl]-acetamide (Compound 4 in Table I).

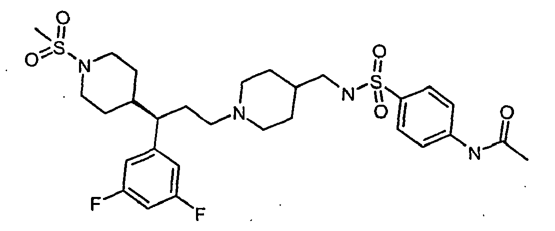

Acetic anhydride (86 mg) was added to a solution of 4-amino-*N*-[(1-{(3*R*)-3-(3,5-difluorophenyl)-3-[1-(methylsulfonyl)piperidin-4-yl]propyl}piperidin-4-yl)methyl]benzenesulfonamide (0.5 g; Example 6) and pyridine (67 mg) in dichloromethane (25 ml) and the mixture was stirred for 2 hours. An additional aliquot of acetic anhydride was added and stirring continued for 16 hours. The reaction mixture was washed with water (25 ml), dried and evaporated to dryness. The residue so obtained was purified by chromatography on silica eluting with a solvent gradient of ethyl acetate: 30% methanol/ethyl acetate and the product was obtained as a white solid after trituration with diethyl ether, yield 146 mg, MH⁺ 626, NMR (CDCl₃): 1.1-2.1 (m, 17H) 2.2 (s, 3H) 2.3-2.6 (m, 3H) 2.7 (s, 3H) 2.8 (m, 2H) 3.7 (m, 1H) 3.8 (m, 1H) 6.6 (m, 3H) 7.6-7.8 (q, 4H) 7.9 (s, 1H).

### Example 9

This Example illustrates the preparation of *N*-[(1-{(3*R*)-3-(3,5-difluorophenyl)-3-[1-(methylsulfonyl)piperidin-4-yl]propyl}piperidin-4-yl)methyl]-4-[(methylsulfonyl)amino]-benzenesulfonamide (Compound 5 in Table I)

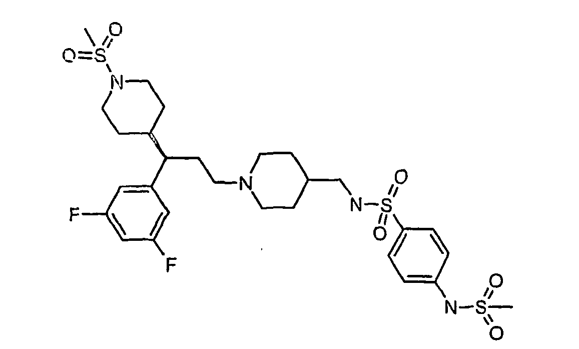

Methanesulphonyl chloride (90 mg) was added to a solution of 4-amino-*N*-[(1-{(3*R*)-3-(3,5-difluorophenyl)-3-[1-(methylsulfonyl)piperidin-4-yl]propyl}piperidin-4-yl)methyl]benzenesulfonamide (0.5 g; Example 6) and pyridine (67 mg) in dichloromethane (25 ml) and the mixture was stirred for 2 hours. An additional portion of methanesulphonyl chloride (30 mg) was added and stirring was continued for 16 hours. The solvent was removed and the residue was purified by chromatography on a 40g Biotage silica column eluting with a solvent gradient of ethyl acetate:25% methanol/ethyl acetate to give an oil which solidified on trituration with diethyl ether, yield 421 mg, MH⁺ 663, NMR (CDCl₃): 1.1-2.2 (m, 12H) 2.8 (s, 3H) 3.1. (s, 3H) 3.4 (m, 3H) 6.9-7.1 (m, 3H) 7.3(d, 2H) 7.6 (t, 1H) 7.7 (d, 2H).

### Example 10

This Example illustrates the preparation of *N-*[(1-{(3*R*)-3-(3,5-difluorophenyl)-3-[4-(methylsulfonyl)phenyl]propyl}piperidin-4-yl)methyl]-3-(1H tetrazol-5-yl)-benzenesulfonamide (Compound 18 in Table II)

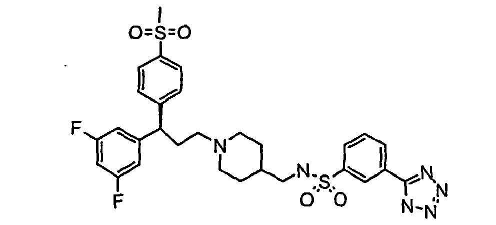

Sodium azide (88 mg) and ammonium chloride (72 mg) were added to a solution of 3-cyano-*N*-[(1-{(3*R*)-3-(3,5-difluorophenyl)-3-[4-(methylsulfonyl)phenyl]propyl}piperidin-4-yl)methyl]benzenesulfonamide (400 mg) in DMF (20 ml) and the mixture was stirred and heated at 100 °C for 8 hours, evaporated to dryness and diluted with water (25 ml). The solid product was dried under vacuum, yield 300 mg, MH⁺ 631, NMR (DMSO d₆): 1.1-1.9 (m, 5H) 2.7 (m, 2H) 2.9 (m, 3H) 3.1 (s, 3H) 4.2 (t, 1H) 7.0-7.2 (m, 3H) 7.6-8 (m, 7H) 8.2 (m, 1H) 8.4 (s, 1H).

### Example 11

This Example describes the preparation of *N*-[(1-{(3*R*)-3-(3,5-difluorophenyl)-3-[4-(methylsulfonyl)phenyl]propyl}piperidin-4-yl)methyl]-4-(1*H*-tetrazol-5-yl)-benzenesulfonamide (Compound 19 in Table II)

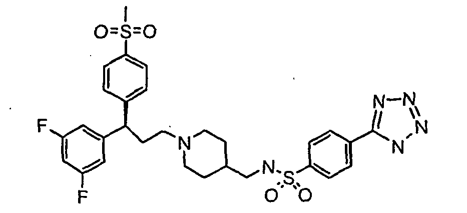

Sodium azide (154 mg) and ammonium chloride (127 mg) were added to a solution of 4-cyano-*N*-[(1-{(3*R*)*-*3-(3,5-difluorophenyl)-3-[4-(methylsulfonyl)phenyl]propyl}piperidin-4-yl)methyl]benzenesulfonamide (700 mg) in DMF (20 ml) and the mixture was stirred and heated at 100 °C for 8 hours, evaporated to dryness and diluted with water (25 ml). The solid product was dried under vacuum at 60 °C, yield 520 mg, MH⁺ 631.

### Method A

### (S)-3-Phenyl-3-(4-methanesulfonylphenyl)propionaldehyde

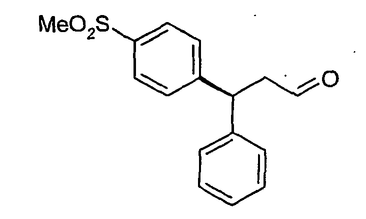

### Step 1: Preparation of (4S,5R)-1-[(S)-3-(4-methanesulfonyl-phenyl)-3-phenyl-propionyl]-3,4-dimethyl-5-phenyl-imidazolidin-2-one

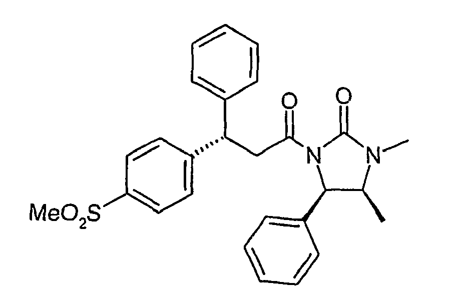

To a mixture of copper (I) iodide (960mg, 5.0mmol) and THF (20mL) was added *N*,*N*,*N*',*N*'-tetramethylethylenediamine (0.83mL, 5.5mmol) and the resulting mixture was stirred at room temperature for 10min. then cooled to -78°C. Phenylmagnesium bromide (5.0mL, 1M in THF, 5.0mmol) was added and the resulting mixture stirred at -78°C for 15min. A solution of di-n-butylboron triflate (3.0mL, 1M in diethyl ether, 3.0mmol) and (E)-(4*S*,5*R*)-1-(3-[4-methanesulfonylphenyl]acryloyl)-3,4-dimethyl-5-phenyl-imidazolidin-2-one (step 4 below), 1.0g, 2.51 mmol) in THF (15mL) was added and the resulting mixture was stirred whilst allowing to warm to room temperature for 18h. The reaction mixture was washed with saturated aqueous ammonium chloride, water and brine, dried (MgSO₄) and evaporated. The residue was purified by eluting through a 20g Bond Elut with gradient of isohexane to ethyl acetate giving the sub-titled compound (1.49g, 100%); NMR (CDCl₃): 0.78 (d, 3H), 2.82 (s, 3H), 3.00 (s, 3H), 3.78 (dd, 1H), 3.80 (m, 1H), 3.98 (dd, 1H), 4.72 (m, 1H), 5.19 (d, 1H), 6.99 (m, 2H), 7.22 (m, 8H), 7.48 (d, 2H), 7.79 (d, 2H); MS: 477 (MH+).

### Step 2: Preparation of (S)-3-phenyl-3-(4-methanesulphonylphenyl)propan-1-ol

To a solution of (4*S*,5*R*)-1-[(*S*)-3-(4-methanesulphonyl-phenyl)-3-phenyl-propionyl]-3,4-dimethyl-5-phenyl-imidazolidin-2-one (846mg, 1.78nunol) in THF (20mL) at 0°C was added lithium aluminium hydride (3.6mL, 1M in THF, 3.6mmol) and the resulting mixture was stirred for 15min. The reaction was quenched by the addition of 2M aqueous sodium hydroxide. The phases were separated and the organic phase pre-absorbed onto a Bond Elut and eluted with a gradient of isohexane to ethyl acetate giving the sub-titled compound as a white solid (285mg, 55%); NMR (GDCl₃): 1.63 (br s, 1H), 2.33 (m, 2H), 3.00 (s, 3H), 3.59 (t, 2H), 4.28 (t, 1H), 7.23 (m, 5H), 7.43 (d, 2H), 7.82 (d, 2H).

### Step 3: Preparation of the title compound

To a solution of (*S*)-3-phenyl-3-(4-methanesulfonylphenyl)propan-1-ol (244mg, 0.84mmol) in DCM (5mL) was added Dess-Martin periodinane (392mg, 0.92mmol) and the resulting mixture was stirred at room temperature for 1.5h. The mixture was washed with 2M aqueous sodium hydroxide (2 x 10mL), dried and evaporated to give the title compound.

### Step 4: Preparation of E-(4S,5R)-1-(3-[4-Methanesulphonylphenyl]acryloyl)-3,4-dimethyl-5-phenyl-imidazolidin-2-one

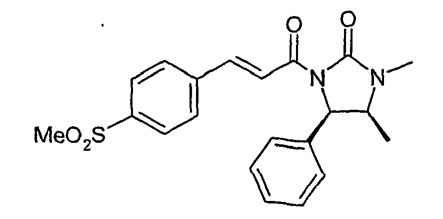

To a stirred solution of 3-(4-methanesulphonylphenyl)acrylic acid (7.14g, 31.5mmol) in DCM (10mL) was added thionyl chloride (3mL, 34.7mmol) dropwise and the resulting mixture was stirred at room temperature for 18h. To this solution was added DIPEA (5.04mL, 28.9mmol) dropwise at room temperature. The resulting solution was added to a stirred solution of (4R, 5S)-1,5-dimethyl-4-phenyl-imidazolidin-2-one (5.0g, 26.3mmol) in DCM (20mL) and DIPEA (4.58mL, 26.9mmol) and the resulting mixture stirred at room temperature for 4h. The mixture was washed with water and brine, pre-absorbed onto a Bond Elut and eluted with a gradient of isohexane to ethyl acetate giving the title compound as a solid (7.61g, 73%); NMR (CDCl₃): 0.84 (d, 3H), 2.89 (s, 3H), 3.04 (s, 3H), 3.98 (m, 1H), 5.42 (d, 1H), 7.20 (m, 2H), 7.32 (m, 3H), 7.69 (d, 1H), 7.74 (d, 2H), 7.93 (d, 2H), 8.31 (d, 1H); MS: 399 (MH+).

### Method B

### 3-Phenyl-3-(N-methanesulphonylpiperidin-4-yl)propionaldehyde

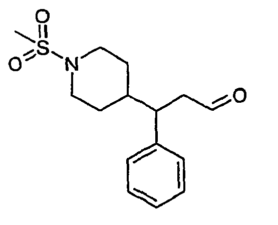

### Step 1 Preparation of 4-benzoyl-1-methanesulphonylpiperidine

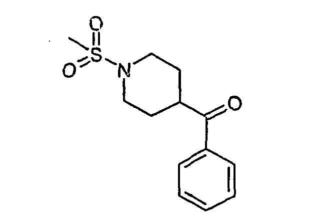

Methanesulphonyl chloride was added to a stirred slurry of 4-benzoylpiperidine hydrochloride (4.51g) and triethylamine (8.35ml) in dichloromethane (100ml) at 0°C. The reaction mixture was allowed to warm to room temperature and was stirred for 16 hours. The mixture was diluted with dichloromethane (50ml) and washed with ammonium chloride solution (2x25ml) and brine (25ml), dried and evaporated to dryness to give 4-benzoyl-1-methanesulphonylpiperidine as a white solid, yield 3.98g. NMR (CDCl₃): 1.93 (m, 4H), 2.81 (s, 3H), 2.98 (d-t, 2H), 3.40 (m, 1H), 3.77 (m, 2H), 7.43 (t, 2H), 7.57 (t, 1H), 7.89 (d, 2H).

### Step 2 Preparation of ethyl 3-phenyl-3-(N-methanesulphonylpiperidin-4-yl)acrylate.

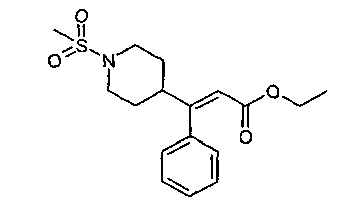

Lithium bis(trimethylsilyl)amide (16.3ml of a 1M solution in THF) was added dropwise to a solution of triethylphosphonoacetate (2.93ml) in THF at 0°C under an argon atmosphere and the mixture was stirred for 30 minutes. A slurry of 4-benzoyl-1-methanesulphonylpiperidine (3.96g) in THF (30ml) was added, the reaction mixture was allowed to warm to room temperature and stirring was continued for 24 hours. The reaction mixture was diluted with dichloromethane (80ml) and water (80ml). The organic layer was washed with water and the combined aqueous extracts were in turn extracted with dichloromethane (50ml). The combined dichloromethane extracts were washed with brine (25ml), dried and evaporated to dryness. The residue was chromatographed on a 90g Biotage column eluted with a solvent gradient (30-5-% ethyl acetate/isohexane to give a less polar fraction (1.62g) and a more polar fraction (0.53g). Both fractions (cis/trans isomers) were combined and used for the next step.

Less polar NMR (CDCl₃): 1.27 (t, 3H), 1.69 (m, 2H), 1.81 (d, 2H), 2.72 (s, 3H), 2.72 (t, 2H), 3.81 (d, 2H), 3.88 (m, 1H), 4.21 (q, 2H), 5.78 (s, 1H), 7.11 (m, 2H), 7.27 (m, 3H).

More polar NMR (CDCl₃): 1.01 (t, 3H), 1.56 (m, 2H), 1.55 (d, 2H),2.31 (m, 1H), 2.63 (t, 2H), 2.74 (s, 3H), 3.83 (d, 2H), 3.92 (q, 3H), 5.82 (s, 1H), 7.04 (d, 2H), 7.30 (m, 3H).

### Step 3 Preparation of ethyl 3-phenyl-3-(N-methanesulphonylpiperidin-4-yl)propionate

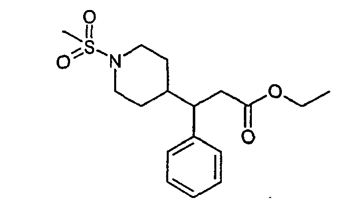

A solution of ethyl 3-phenyl-3-(N-methanesulphonylpiperidin-4-yl)acrylate (2.06g) in ethanol (30ml) was hydrogenated over 24 hours under a hydrogen filled balloon using 20% palladium hydroxide as catalyst. The reaction mixture was filtered through Celite and the filtrate evaporated to dryness. The product obtained was used for the next step without further purification. MH⁺340.

### Step 4 3-Phenyl-3-(N-methanesulphonylpiperidin-4-yl)propan-1-ol.

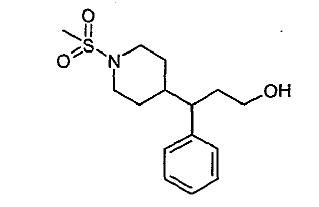

A solution of ethyl 3-phenyl-3-(N-methanesulphonylpiperidin-4-yl)propionate (2g) in THF (10ml) was added to a suspension of lithium aluminium hydride (232mg) in THF (20ml) at 0°C under argon over 30 minutes. The reaction mixture was allowed to warm to room temperature and stirred for 2 hours. Water (10ml) was added followed by magnesium sulphate (10g). The reaction mixture was filtered and the filtrate evaporated to dryness to give the product as a white foam, yield 1.57g. NMR (CDCl₃): 1.40 (m, 4H), 1.57 (m, 1H), 1.78 (m, 1H), 2.01 (m, 2H), 2.45 (m, 2H), 2.58 (t, 1H), 2.70 (m, 3H), 3.31 (m, 1H), 3.42 (m, 1H), 3.67 (d, 1H), 3.80 (d, 1H), 7.04 (d, 1H), 7.19 (t, 1H), 7.29 (q, 2H).

### Step 5 Preparation of the title compound

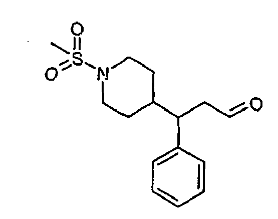

Dess-Martin periodinane (739mg) was added to a stirred solution of 3-phenyl-3-(N-methanesulphonylpiperidin-4-yl)propan-1-ol (454mg) in dichloromethane (8ml) and stirring was continued for 2 hours. The reaction mixture was diluted with dichloromethane (100ml) and washed with 2M sodium hydroxide (2x50ml), brine (50ml) and dried. The product obtained on removal of the solvent was used in subsequent steps without purification.

### Method C

### (R)-3-(3,5-Difluorophenyl)-3-(4-methanesulfonylphenyl)propionaldehdye

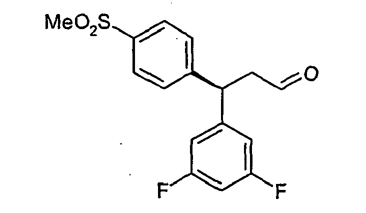

This was prepared from (4*S*,5*R*)-1-(3-[4-methanesulfonylphenyl]acryloyl)-3,4-dimethyl-5-phenyl-imidazolidin-2-one and 3,5-difluorophenylmagnesium bromide using a method similar to that used to prepare (*S*)-3-phenyl-3-(4-methanesulfonylphenyl)propionaldehyde from phenylmagnesium bromide (Method A); NMR (CDCl₃): 3.05 (s, 3H), 3.20 (d, 2H), 4.72 (t, 1H), 6.75 (m, 3H), 7.35 (d, 2H), 7.88 (d, 2H), 9.75 (s, 1H).

### Method D

### (R) 3-(1-Methanesulphonylpiperidin-4-yl)-3-[3,5-difluorophenyl]propionaldehyde

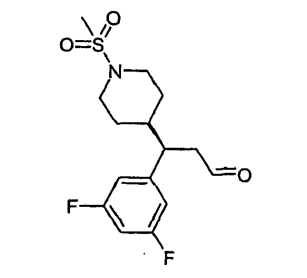

### Step 1 3-[N-(benzyloxycarbonylpiperidin-4-yl)]propenoic acid

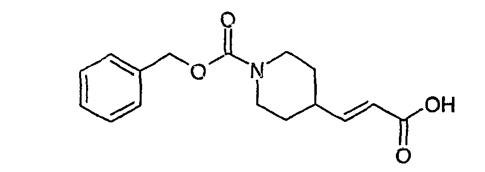

A mixture of N-benzyloxycarbonyl-4-formylpiperidine (10g), malonic acid (4.2), pyridine (4 ml) and piperidine (0.4 ml) was heated at 100°C for 2 hours. The reaction mixture was allowed to cool and was diluted with ethyl acetate (100 ml). The solution was washed with 2M HCl (2X100 ml), dried and evaporated to dryness. The residue was triturated with isohexane to give the title compound, yield 13.5g. NMR (AMSOd6) : 1.2 (m, 2H) 1.7 (m, 2H) 2.35 (m, 1H) 2.85 (m, 2H) 4 (d, 2H) 5.05 (s, 2H) 5.75 (d, 1H) 6.75 (m, 1H) 7.35 (m, 5H) 12.25 (broad peak, 1H)

### Step 2 N-(benzyloxycarbonylpiperidin-4-yl)propenoic acid isopropyl ester

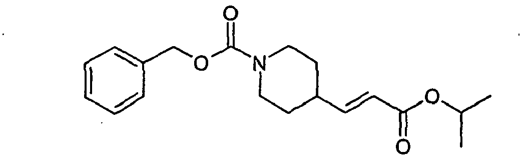

A solution of N-(benzyloxycarbonylpiperidin-4-yl)propenoic acid (52g) in isopropanol (500 ml) containing concentrated sulphuric acid (20 ml) was heated under reflux for 32 hours. The solvent was evaporated and the residue was dissolved in ethyl acetate (250 ml). The ethyl acetate solution was washed with water (2X250 ml) and saturated aqueous sodium bicarbonate (2X25 ml) and dried. The residue obtained on evaporation of the solvent was chromatographed on a Bond Elut cartridge eluted with a solvent gradient (isohexane-25% ethyl acetate/isohexane) to give the title compound, yield 54g.

### Step 3 Preparation of (R) 3-(N-benzyloxycarbonylpiperidin-4-yl)-3-(3,5-difluorophenyl)propanoic acid isopropyl ester

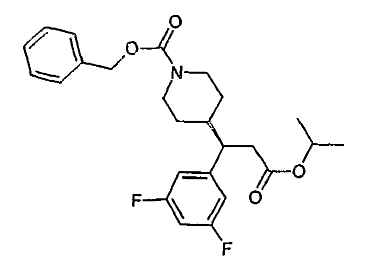

Dioxane (100 ml) was charged to a 500 ml three necked flask and purged with argon for 10 minutes. Acetylacetonatobis[ethylene]rhodium (I) (620 mg) and R-BINAP were added and the mixture was stirred for 10 minutes. 3,5-Difluorophenylboronic acid (19g) was added and the mixture was purged with argon for 10 minutes. N-(benzyloxycarbonylpiperidin-4-yl)propenoic acid isopropyl ester (8 g) and ethanediol (20 ml) in dioxane (100 ml) were added and the mixture was purged with argon for 10 minutes. The mixture was heated at 100°C for 18 hours, allowed to cool and was passed through activated alumina (200g) washed through with ethyl acetate (3X100 ml). The combined washings were evaporated to dryness and the residue obtained was dissolved in ethyl acetate (100 ml) and washed successively with saturated aqueous sodium bicarbonate (2X100 ml) and 2M HCl (2X100 ml), dried and evaporated to dryness. The product obtained (12g) was shown to be 40% of the required material by NMR and was used without further purification in the subsequent reactions.

### Step 4 Preparation of (R) 3-(piperidin-4-yl)-3-(3,5-difluorophenyl)propanoic acid isopropyl ester.

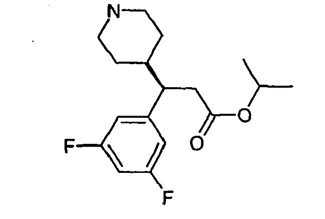

A solution of (R) 3-(N-benzyloxycarbonylpiperidin-4-yl)-3-(3,5-difluorophenyl)propanoic acid isopropyl ester (12g) in ethanol (300 ml) containing 20% palladium hydroxide on charcoal (2g) was hydrogenated under a hydrogen filled balloon. The catalyst was filtered and the filtrate was evaporated to dryness to give the title compound (10g) which was used without further purification.

### Step 5 Preparation of(R) 3-(N-methanesulphonylpiperidin-4-yl)-3-(3,5-difluorophenyl)-propanoic acid isopropyl ester.

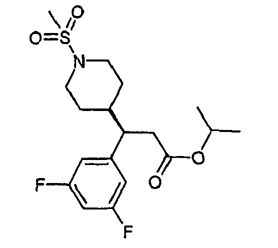

Methanesulphonyl chloride (3.7g) was added to a solution of (R) 3-(piperidin-4-yl)-3-(3,5-difluorophenyl)propanoic acid isopropyl ester (10g) and triethylamine (3.89g) in dichloromethane (100 ml) at 0°C. The reaction mixture was allowed to warm to room temperature and was washed with 2M HCI (2X50 ml) and saturated aqueous sodium bicarbonate (2X50 ml), dried and evaporated to dryness to give the title compound (10g) which was used without further purification.

### Step 6 Preparation of (R) 3-(N-methanesulphonylpiperidin-4-yl)-3-(3,5-difluorophenyl)propanol

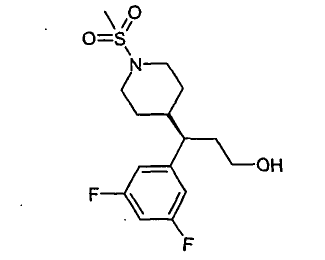

Lithium aluminium hydride (25 ml of a 1M solution in THF) was added dropwise over 15 minutes to a solution of (R) 3-(N-methanesulphonylpiperidin-4-yl)-3-(3,5-difluorophenyl)propanoic acid isopropyl ester (10g) in THF (150 ml) at -10°C. The reaction mixture was stirred at -10°C for 30 minutes, 2M NaOH (25 ml) was added, the mixture was filtered and the filtrate evaporated to dryness. The residue obtained was dissolved in ethyl acetate and washed with 2M HCl (2X100 ml) and dried. The residue obtained on removal of the solvent was chromatographed on a Bond Elut column eluting with a solvent gradient (80% ethyl acetate/isohexane-ethyl acetate) to give the title compound, yield 2.2 g. NMR (Dimethylsulfoxide d6): 0.95-1.2 (m, 2H) 1.3 (m, 1H) 1.6 (m.2H) 1.9 (m, 2H) 2.6 (m, 2H) 2.8 (s, 3H) 3.1 (m, 1H) 3.2 (m, 1H) 3.4 (m, 1H) 3.5 (m, 1H) 6.8-7 (m, 3).

### Step 7 Preparation of (R) 3-(N-methanesulphonylpiperidin-4-yl)-3-(3,5-difluorophenyl)propionaldehyde.

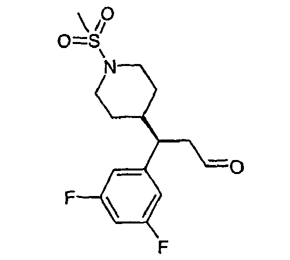

Dess-Martin periodinane (1g) was added to a solution of (R) 3-(N-methanesulphonylpiperidin-4-yl)-3-(3,5-difluorophenyl)propanol (0.8g) in dichloromethane (40 ml) and the mixture was stirred for 1.5 hours. The reaction mixture was washed with 2M NaOH (2X20 ml) and dried. The solution of the title compound in dichloromethane was used in subsequent reactions.

### Method E

### (R) 3-(N-methanesulphonylpiperidin-4-yl)-3-phenylpropanol

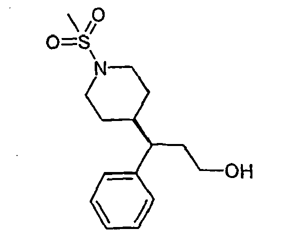

### Step 1 Preparation of 3-(N-methanesulphonylpiperidin-4-yl)propenoic acid acid chloride.

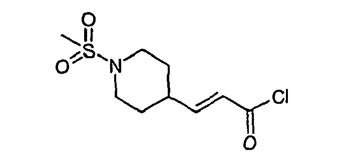

1-Chloro-N,N,2-trimethylpropenylamine (1.06 ml) was added dropwise over 10 minutes to a suspension of 3-(N-methanesulphonylpiperidin-4-yl)propenoic acid (1.86g, prepared from N-methanesulphonylpiperidine-4-carboxaldehyde [CAS 241134-3 5-0] according to step 1 of Method C) in THF (20 ml) under an atmosphere of argon and the mixture was stirred for 2 hours and used directly in step 2.

### Step 2 Preparation of 1-[3-(N-methanesulphonylpiperidin-4-yl)propenyl]-(4S, 5R)-3,4-dimethyl-4-phenyl-imidazolidin-2-one.

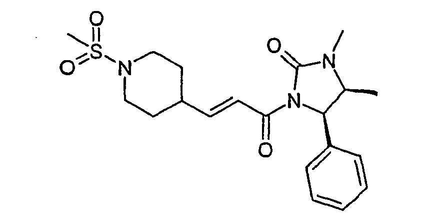

Lithium bis(trimethylsilyl)amide (8 ml of a 1M solution in THF) was added dropwise to a suspension of (4R,5S)-1,5-dimethyl-4-phenyl-2-imidazolidinone (1.52g) in THF (20 ml) under argon at -10°C. the reaction mixture was stirred at -10°C for 10 minutes, allowed to warm to 0°C and maintained at this temperature for 10 minutes then cooled again to -10°C. The solution of the acid chloride prepared in Step 1 was added dropwise and the reaction mixture was allowed to warm to room temperature and washed with water (100 ml). The aqueous extract was extracted with ethyl acetate (3X50 ml) and the ethyl acetate extracts were dried and the residue passed through a 90g Biotage column eluting with a solvent gradient (50% ethyl acetate/isohexane-70% ethyl acetate/isohexane). Yield 1.89g. LC-MS MH⁺ 406, NMR (CDCl₃): 0.8 (d, 3H) 1.5-1.6 (m, 3H) 1.9 (m, 2H) 2.3 (m, 1H), 2.7 (m, 2H) 2.75 (s, 3H) 2.8 (s, 3H) 3.75 (m, 2H) 3.9 (m, 1H) 5.3 (d, 1H) 6.85 (d-d, 1H) 7.1 (d, 1H) 7.2-7.35 (m, 3H) 7.45 (d, 1H).

### Step 3 Preparation of (R) 1-[3-phenyl-3-(methanesulphonylpiperidin-4-yl)propionyl]-(4S,SR)-3,4-dimethyl-5-phenyl-imidazolidin-2-one.

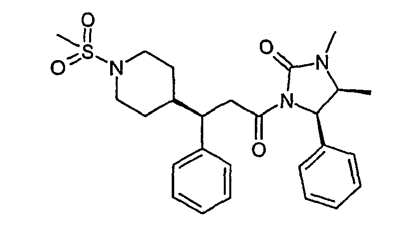

A mixture of copper(I) iodide (1.78 g) and *N,N,N'N*'-tetramethylethylenediamine (1.41 ml) in THF (50 ml) was stirred under argon for 1 hour then cooled to -78°C and phenylmagnesium bromide (5.4 ml of a 1M solution in THF) was added and the mixture was stirred at -78 °C for 30 minutes. A solution of 1-[3-(N-methanesulphonylpiperidin-4-yl)propenyl]-(4S, 5R)-3,4-dimethyl-5-phenyl-imidazolidin-2-one (1.89g) and dibutylboron triflate (4.67 ml of a 1M solution in diethylether in THF (50 ml) was added over 10 minutes and the reaction mixture was stirred at -78°C for 1 hour then allowed to warm to room temperature. The reaction mixture was concentrated and filtered through a pad of silica (50g) washed with ethyl acetate (2X50 ml) and the ethyl acetate washings were washed with 2M HCl (2X150 ml) and dried. The residue obtained on removal of the solvent was passed through a 90g Biotage column eluting with a solvent gradient (50% ethyl acetate/isohexane-70% ethyl acetate/isohexane) to give the product as a yellow solid, yield 1.34g, MH⁺ 484. NMR (CDCl₃): 0.7 (d, 3H) 1.2 (m, 1H) 1.35 (m, 1H) 1.5 (m, 1H) 1.9 (m, 1H) 2.45 (m, 1H) 2.55 (m, 1H) 2.7 (s, 3H) 2.8 (s, 3H) 3.1 (m, 1H) 3.2 (d-d, 1H) 3.4 (m, 1H) 3.65 (m, 1H) 3.75-3.9 (m, 3H) 5.2 (d, 1H) 6.7 (d, 2H) 7.05-7.25 (m, 8H).

### Step 4 Preparation of the title compound

A solution of (R) 1-[3-phenyl-3-(methanesulphonylpiperidin-4-yl)propionyl]-(4S,5R)-3,4-dimethyl-5-phenyl-imidazolidin-2-one (1.34g) in THF (14 ml) was added to a solution of lithium aluminium hydride (2.77 ml of a 1M solution in THF) in THF (10 ml) at 0°C and the mixture was allowed to warm to room temperature over 1 hour. Water (5 ml) was added cautiously, then THF (15 ml) and solid magnesium sulphate. The reaction mixture was filtered and the filtrate was passed through a 40 g Biotage column eluted with a solvent gradient (50% ethyl acetate/isohexane-70% ethyl acetate/isohexane) to give the title compound as a white solid, yield 338 mg. NMR (CDCl₃) : 1.15-1.25 (m, 2H) 1.3-1.5 (m, 2H) 1.6 (m, 1H) 1.75 (m, 1H) 1.95-2.10 (m, 2H) 2.5 (m, 2H) 2.6 (m, 1H) 2.7 (s, 3H) 3.3-3.4 (m, 2H) 3.45 (m, 1H) 3.7 (m, 1H) 3.85 (m, 1H) 7.05 (m, 2H) 7.15-7.35 (m, 3H).

### Method F

### (S) 3-phenyl-3-(tert-butoxycarbonylamino)propionaldehyde

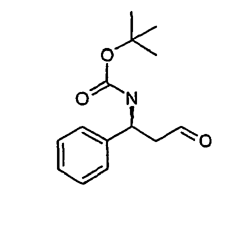

Lithium aluminium hydride (19 ml of 1M solution in THF) was added to a solution of (S) 3-phenyl-3-(tert-butoxycarbonylamino)propionic acid (5.01g) in THF (50ml) at 0°C. The reaction mixture was stirred for 1 hour and ethyl acetate (20ml) was added followed by water (0.5ml), 6M sodium hydroxide (0.5ml) and water (5ml). The mixture was filtered through Celite and evaporated to dryness to give (S) 3-phenyl-3-(tert-butoxycarbonylamino)propanol, yield 2.89g. This material was dissolved in dichloromethane (40ml) and Dess Martin periodinane (2.12g) was added. The reaction mixture was stirred for 1 hour then washed with 2M sodium hydroxide (2x20ml) and brine (10ml) and dried. The dichloromethane solution was concentrated to a volume of about 20ml and used directly in the next stage.

### Method G

### Preparation of 1-(1-{(3R)-3-(3,5-difluorophenyl)-3-[1-(methylsulfonyl)piperidin-4-yl]propyl}piperidin-4-yl)methanamine

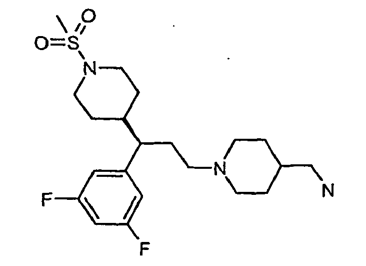

### Step 1 Preparation of tert-butyl [(1-{(3R)-3-(3,5-difluorophenyl)-3-[1-(methylsulfonyl)-piperidin-4-yl]propyl}piperidin-4-yl)methyl]carbamate

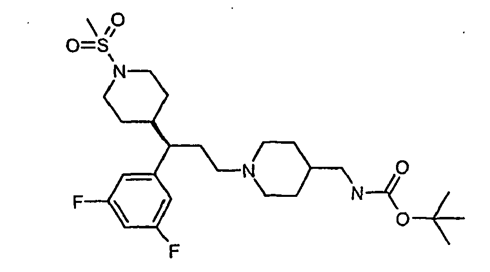

Sodium triacetoxyborohydride (1.26 g) was added to a solution of (3R)-3-(3,5-difluorophenyl)-3-[1-(methylsulfonyl)piperidin-4-yl]propanal (2.0 g) and *tert*-butyl (piperidin-4-ylmethyl)carbamate (1.26 g) in dichloromethane (75 ml) and the mixture was stirred for 4 hours then washed with 2M NaOH (2x50 ml), dried and evaporated to dryness. The residue obtained on removal of the solvent was purified by chromatography on silica eluting with a solvent gradient of ethyl acetate: 20% methanol/ethyl acetate. The oily poduct obtained gave a solid on trituration with diethyl ether, yield 3g, MH⁺ 530.

### Step 2 Preparation of the title compound

A solution of *tert-*butyl (piperidin-4-ylmethyl)carbamate (3 g) in 4M HCl in dioxane (25 ml) and methanol (5 ml) was stirred for 2 hours. Diethyl ether (100 ml) was added and the solid obtained on filtration was dissolved in water (50 ml). The aqueous solution was basified with 2M NaOH and extracted with dichloromethane (5x25 ml). The combined dichloromethane extracts were dried and the gum obtained on removal of the solvent was stirred with diethyl ether to give the title compound as a white solid, yield 2.1 g, MH⁺ 429. NMR (CDCl₃): 1.1-2.7 (m, 21H) 2.75 (s, 3H) 2.9 (m, 2H) 3.6-3.9 (m, 4H) 6.6 (m, 3H).

### Method H

### Preparation of [2-(1-{(3R)-3-(3,5-difluorophenyl)-3-[4-(methylsulfonyl)phenyl]propyl}piperidin-4-yl)ethyl]amine

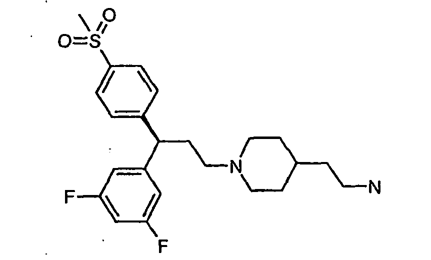

### Step 1 Preparation of tert-butyl [2-(1-{(3R)-3-(3,5-difluorophenyl)-3-[4-(methylsulfonyl)-phenyl]propyl}piperidin-4-yl)ethyl]carbamate

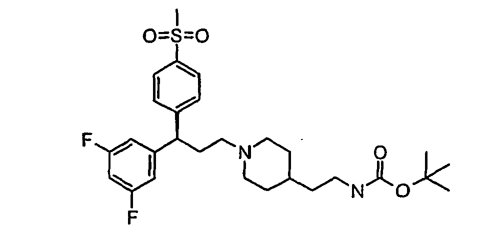

Sodium triacetoxyborohydride (2.78 g) was added to a solution of (3R)-3-(3,5-difluorophenyl)-3-[4-(methylsulfonyl)phenyl]propanal (4.2 g) and *tert*-butyl 4-piperidin-4-ylbutanoate (3 g) in dichloromethane (100 ml) and the mixture was stirred for 16 hours. The reaction mixture was washed with 2M NaOH (2x50 ml), dried and poured onto a 50g SCX2 cartridge and eluted with methanol (5x20 ml) and 1M ammonia in methanol (6x20 ml). The methanolic ammonia washings were evaporated to dryness to give *tert*-butyl [2-(1-{(3R)-3-(3,5-difluorophenyl)-3-[4-(methylsulfonyl)phenyl]propyl}piperidin-4-yl)ethyl]carbamate, yield 5.6g, MH⁺ 537, NMR (CDCl₃): 1.3 (m, 3H) 1.4 (m, 5H) 1.45 (s, 9H) 1.7 (m, 3H) 1.85 (m, 2H) 2.7 (m, 2H) 3.05 (s, 3H) 3.15 (m, 2H) 4.1 (m, 1H) 4.4 (Br m, 1H) 6.6-6.8 (m, 3H) 7.4 (d, 2H) 7.9 (d, 2H)

### Step 2 Preparation of the title compound

4M HCl in dioxane (40 ml) was added to a paste of *tert-*butyl [2-(1-{(3*R*)-3-(3,5-difluorophenyl)-3-[4-(methylsulfonyl)phenyl]propyl}piperidin-4-yl)ethyl]carbamate (5.6 g) in dioxane (5 ml) and the mixture was stirred for 1.5 hours. The solid was filtered and dissolved in water (50 ml). The aqueous soltion was basified with 2M NaOH and was extracted with dichloromethane (5x50 ml). The combined extracts were dried and evaporated to dryness to give the title compound, yield 4.2g, MH⁺ 437, NMR (CDCl₃): 1.1-1.5 (m, 9H) 1.7 (d, 2H) 1.9 (t, 2H) 2.7 (t, 2H) 2.8 (m, 2H) 3.05 (s, 3H) 4.1 (m, 1H) 6.6-8 (m, 3H) 7.4 (d, 2H) 7.9 (d, 2H).

### Example 12

The ability of compounds to inhibit the binding of MIP-1α was assessed by an *in vitro* radioligand binding assay. Membranes were prepared from Chinese hamster ovary cells which expressed the recombinant human CCR5 receptor. These membranes were incubated with 0.1nM iodinated MIP-1α, scintillation proximity beads and various concentrations of the compounds of the invention in 96-well plates. The amount of iodinated MIP-1α bound to the receptor was determined by scintillation counting. Competition curves were obtained for compounds and the concentration of compound which displaced 50% of bound iodinated MIP-1α was calculated (IC₅₀). Certain compounds of formula (I) have an IC₅₀ of less than 50µM.

Results from this test for certain compounds of the invention are presented in Table VI. In Table VI the results are presented as Pic50 values. A Pic50 value is the negative log (to base 10) of the IC₅₀ result, so an IC50 of 1µM (that is 1 x 10⁻⁶M) gives a Pic50 of 6. If a compound was tested more than once then the data below is an average of the probative tests results.

**TABLE VI**

| Table Number | Compound number | Pic50 |
|---|---|---|
| I | 1 | 9.3 |
| I | 4 | 8.1 |
| I | 7 | 8.3 |
| II | 6 | 6.8 |
| II | 12 | 7.7 |
| II | 13 | 8.0 |
| II | 16 | 7.3 |
| II | 19 | 8.1 |
| III | 2 | 6.8 |

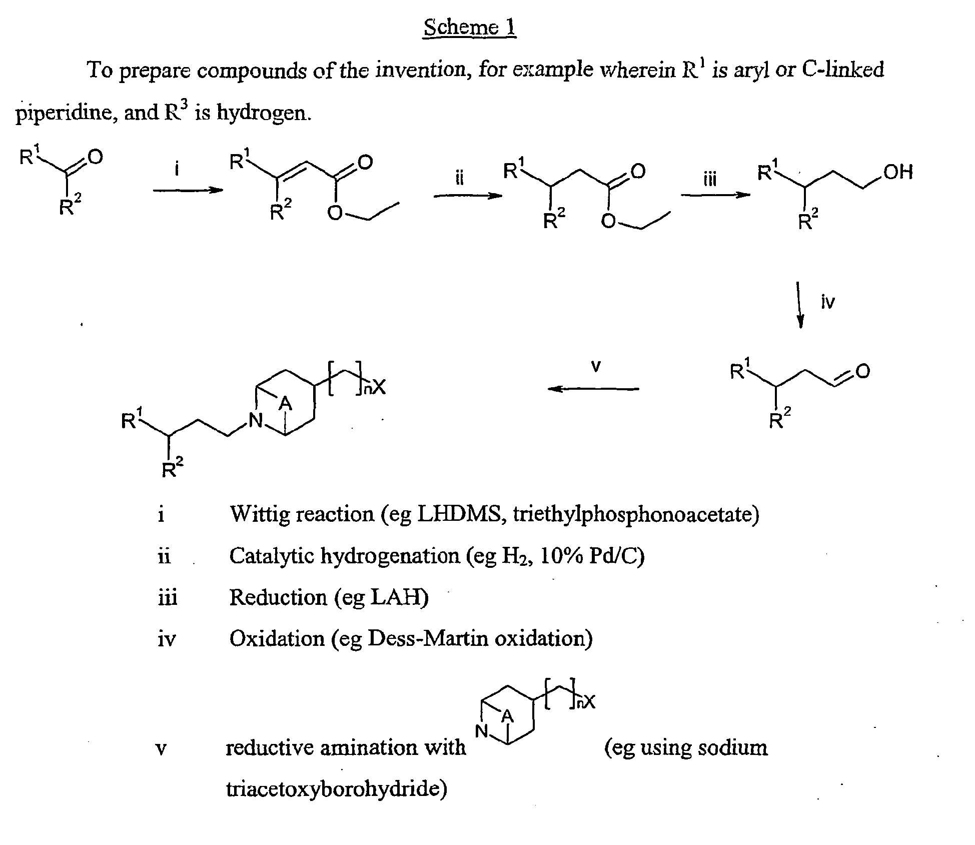

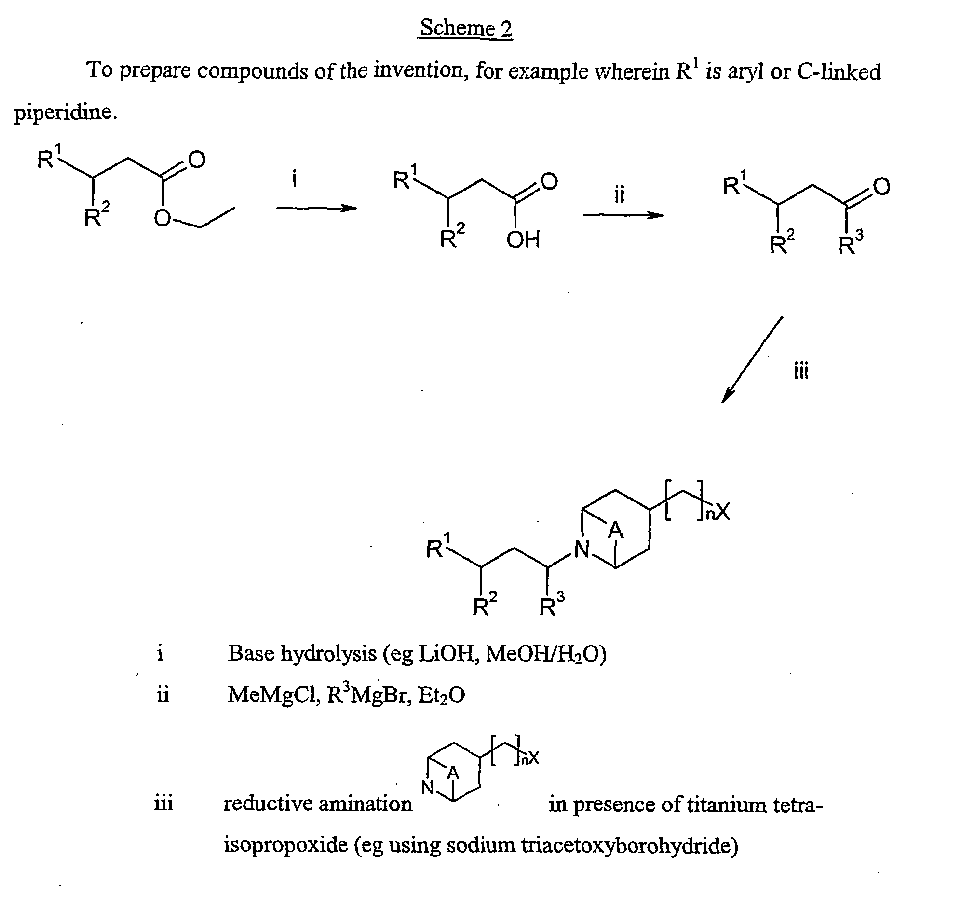

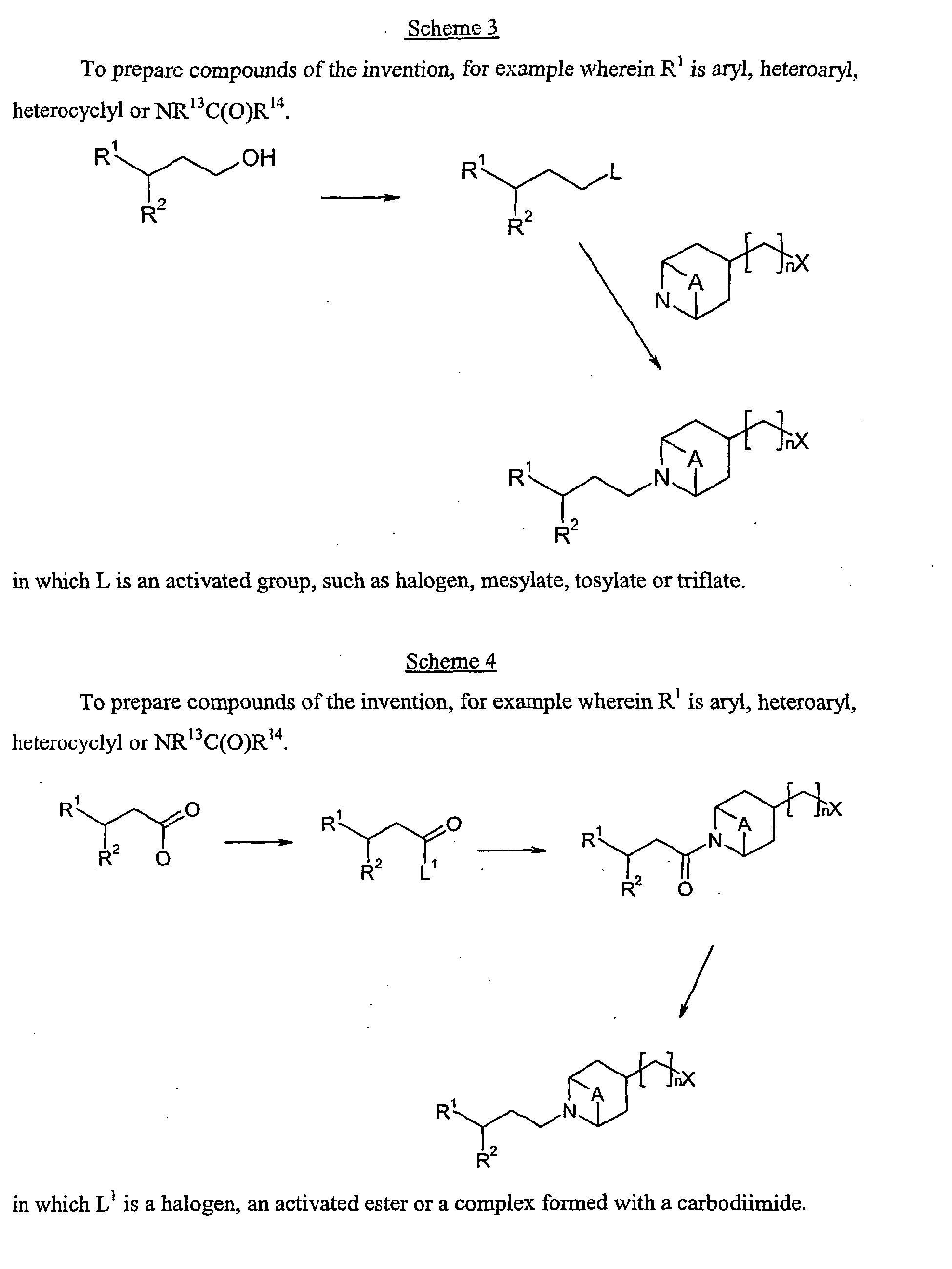

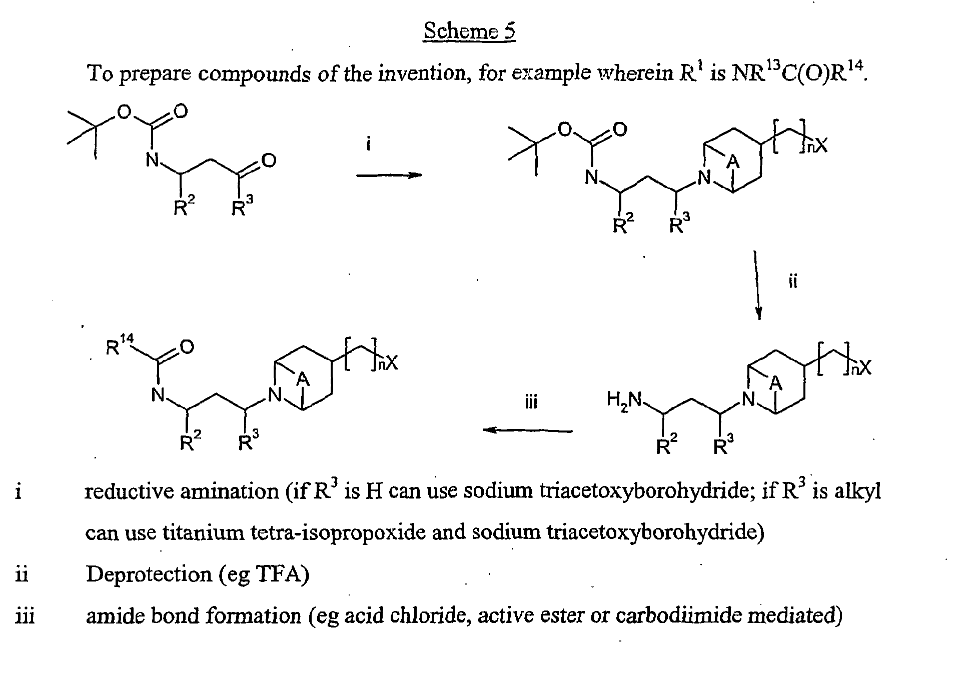

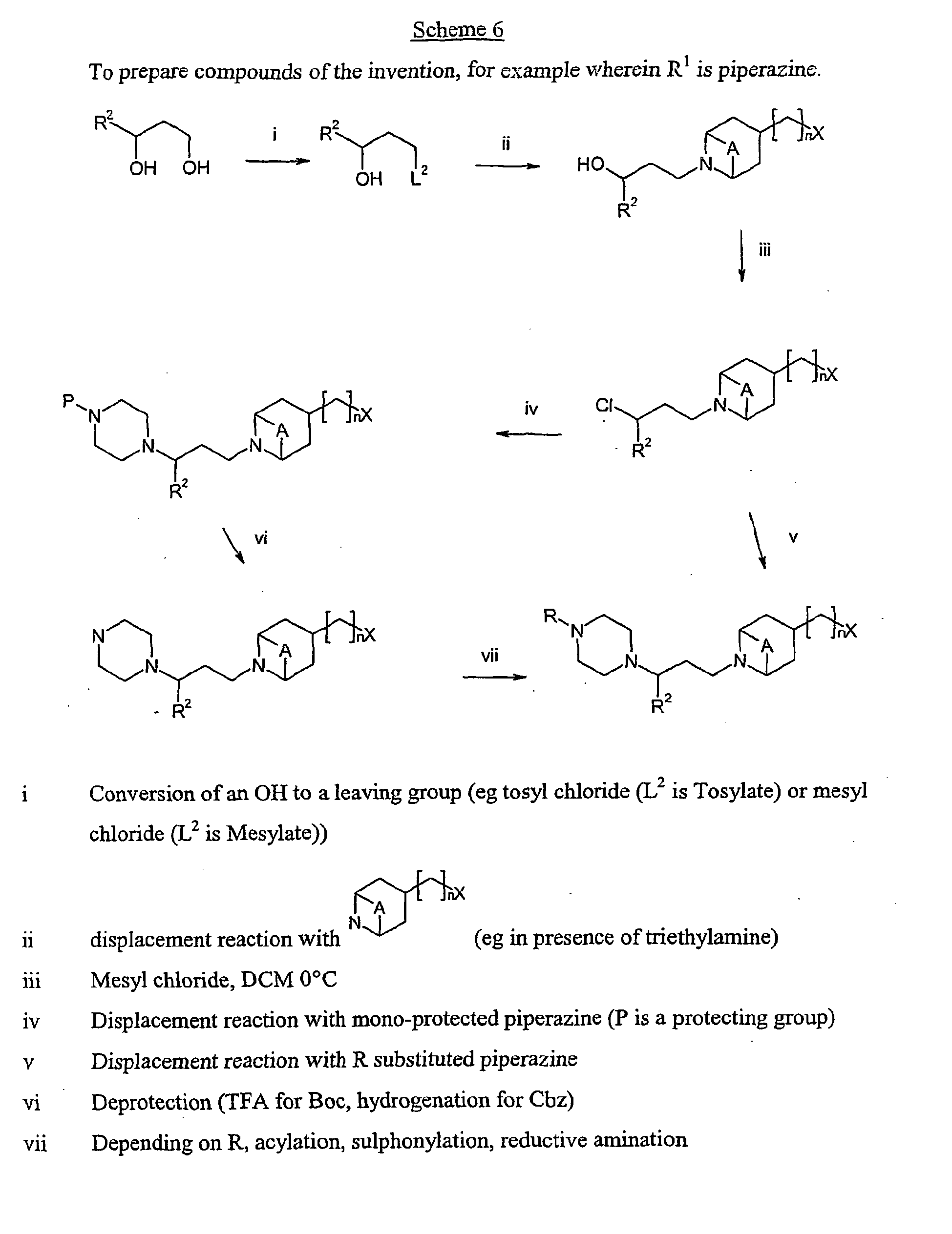

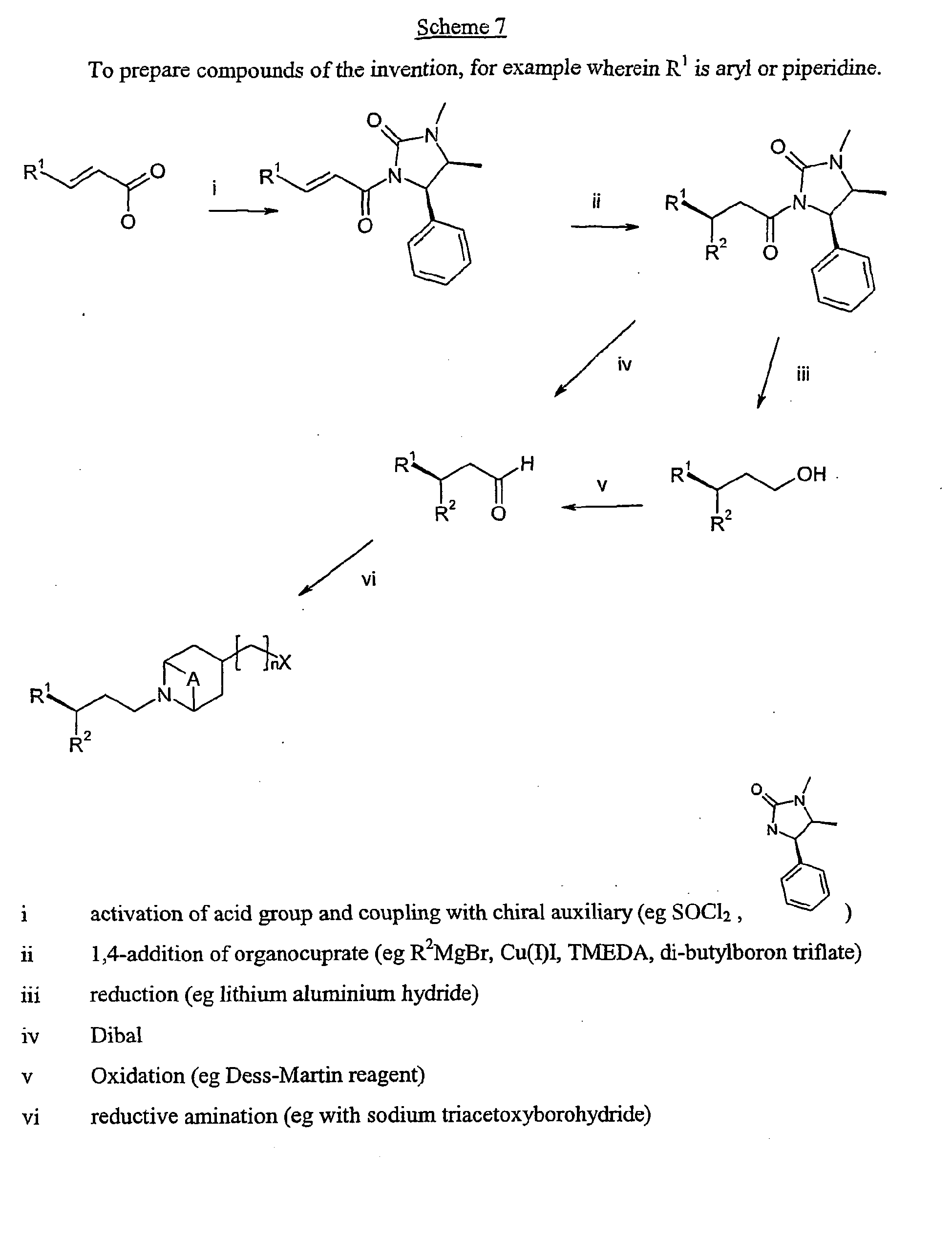

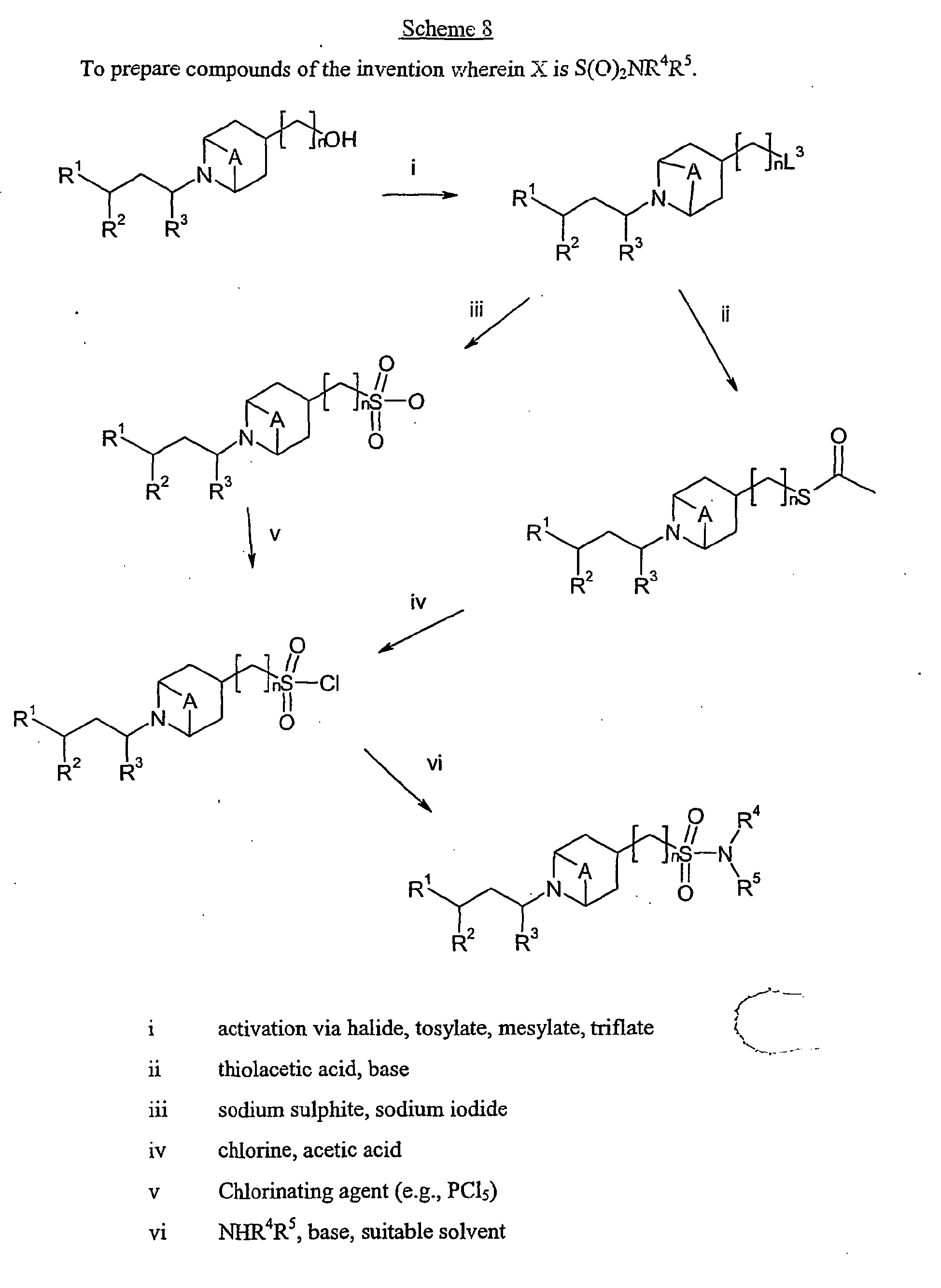

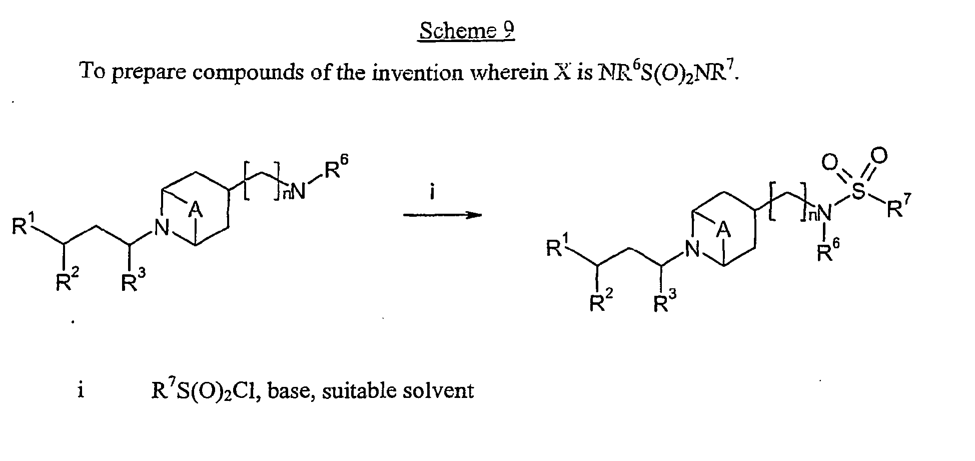

## Claims

1. A compound of formula (I): 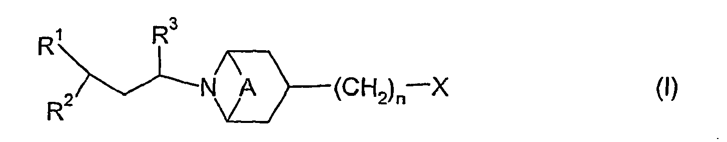 wherein:
A is absent or is (CH₂)₂;
R¹ is C(O)NR¹⁰R¹¹, C(O)₂R¹², NR¹³C(O)R¹⁴, NR¹⁵C(O)NR¹⁶R¹⁷, NR¹⁸C(O)₂R¹⁹, heterocyclyl (for example piperidine, piperazine, pyrrolidine or azetidine), aryl, cycloalkyl or heteroaryl;
R¹⁰, R¹³, R¹⁵, R¹⁶ and R¹⁸ are hydrogen or C₁₋₆ alkyl;
R¹¹, R¹², R¹⁴, R¹⁷ and R¹⁹ are C₁₋₈ alkyl (optionally substituted by halo, hydroxy, C₁₋₆ alkoxy, C₁₋₆ haloalkoxy, C₃₋₆ cycloalkyl (optionally substituted by halo), C₅₋₆ cycloalkenyl, S(C₁₋₄ alkyl), S(O)(C₁₋₄ alkyl), S(O)₂(C₁₋₄ alkyl), heteroaryl, aryl, heteroaryloxy or aryloxy), aryl, heteroaryl, C₃₋₇ cycloalkyl (optionally substituted by halo or C₁₋₄ alkyl), C₄₋₇ cycloalkyl fused to a phenyl ring, C₅₋₇ cycloalkenyl, or, heterocyclyl (itself optionally substituted by oxo, C(O)(C₁₋₆ alkyl), S(O)ₖ(C₁₋₆ alkyl), halo or C₁₋₄ alkyl); or R¹¹,R¹² , R¹⁴ and R¹⁷ can also be hydrogen;
or R¹⁰ and R¹¹, and/or R¹⁶ and R¹⁷ may join to form a 4-, 5- or 6-membered ring which optionally includes a nitrogen, oxygen or sulphur atom, said ring being optionally substituted by C₁₋₆ alkyl, S(O)₁(C₁₋₆ alkyl) or C(O)(C₁₋₆ alkyl);
R² is phenyl, heteroaryl or C₃₋₇ cycloalkyl;
R³ is H or C₁₋₄ alkyl;
X is S(O)₂NR⁴R⁵ or NR⁶S(O)₂R⁷;
R⁷ is aryl, heteroaryl, C₁₋₆ alkyl, C₃₋₇ cycloalkyl, heterocyclyl or NR⁸R⁹ wherein NR⁸R⁹ can be cyclized to form a 4-, 5- or 6-membered ring which optionally includes a nitrogen, oxygen or sulphur atom, said ring being optionally substituted by C₁₋₆ alkyl, S(O)p(C₁₋₆ alkyl) or C(O)(C₁₋₆ alkyl);
R⁴ and R⁸ are aryl, heteroaryl, C₁₋₆ alkyl (optionally substituted by hydroxy or C₁₋₆ alkoxy), C₃₋₇ cycloalkyl or heterocyclyl;
R⁵, R⁶ and R⁹ are, independently, hydrogen or C₁₋₆ alkyl;
n is 1, 2 or 3;
aryl, phenyl and heteroaryl moieties are independently optionally substituted by one or more of halo, cyano, nitro, hydroxy, OC(O)NR²⁰R²¹, NR²²R²³, NR²⁴C(O)R²⁵,
NR²⁶C(O)NR²⁷R²⁸, S(O)₂NR²⁹R³⁰, NR³¹S(O)₂R³², C(O)NR³³R³⁴, CO₂R³⁶,
NR³⁷CO₂R³⁸, S(O)qR³⁹, OS(O)₂R⁴⁹, C₁₋₆ alkyl (optionally mono-substituted by S(O)₂R⁵⁰ or C(O)NR⁵¹R⁵²), C₂₋₆ alkenyl, C₂₋₆ alkynyl, C₃₋₁₀ cycloalkyl, C₁₋₆ haloalkyl, C₁₋₆ alkoxy(C₁₋₆)alkyl, C₁₋₆ alkoxy, C₁₋₆ haloalkoxy, phenyl, phenyl(C₁₋₄)alkyl, phenoxy, phenylthio, phenylS(O), phenylS(O)₂, phenyl(C₁₋₄)alkoxy, heteroaryl, heteroaryl(C₁₋₄)alkyl, heteroaryloxy or heteroaryl(C₁₋₄)alkoxy; wherein any of the immediately foregoing phenyl and heteroaryl moieties are optionally substituted with halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), CF₃ or OCF₃;
unless otherwise stated heterocyclyl is optionally substituted by C₁₋₆ alkyl [optionally substituted by phenyl {which itself optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio,
S(O)(C₁₋₄ alkyl) or S(O)₂(C₁₋₄ alkyl)} or heteroaryl {which itself optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}], phenyl {optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}, heteroaryl {optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}, S(O)₂NR⁴⁰R⁴¹, C(O)R⁴², C(O)₂(C₁₋₆ alkyl) (such as tert-butoxycarbonyl), C(O)₂(phenyl(C₁₋₂ alkyl))(such as benzyloxycarbonyl), C(O)NHR⁴³, S(O)₂R⁴⁴, NHS(O)₂NHR⁴⁵, NHC(O)R⁴⁶,
NHC(O)NHR⁴⁷ or NHS(O)₂R⁴⁸, provided none of these last four substituents is linked to a ring nitrogen;
k,1, p and q are, independently, 0, 1 or 2;
R²⁰, R²², R²⁴, R²⁶, R²⁷, R²⁹, R³¹, R³³, R³⁷, R⁴⁰ and R⁵¹ are, independently, hydrogen or C₁₋₆ alkyl;
R²¹, R²³ R²⁵, R²⁸, R³⁰, R³², R³⁴, R³⁶, R³⁸, R³⁹, R⁴¹, R⁴², R⁴³, R⁴⁴, R⁴⁵, R⁴⁶, R⁴⁷, R⁴⁸,
R⁴⁹, R⁵⁰ and R⁵² are, independently, C₁₋₆ alkyl (optionally substituted by halo, hydroxy, C₁₋₆ alkoxy, C₁₋₆ haloalkoxy, C₃₋₆ cycloalkyl, C₅₋₆ cycloalkenyl, S(C₁₋₄ alkyl), S(O)(C₁₋₄ alkyl), S(O)₂(C₁₋₄ alkyl), heteroaryl, phenyl, heteroaryloxy or phenyloxy), C₃₋₇ cycloalkyl, phenyl or heteroaryl; wherein any of the immediately foregoing phenyl and heteroaryl moieties are optionally substituted with halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃;
R²¹, R²³, R²⁵, R²⁸, R³⁰, R³⁴, R³⁵, R³⁶, R⁴¹, R⁴², R⁴³, R⁴⁵, R⁴⁶, R⁴⁷ and R⁵² may additionally be hydrogen;
or a pharmaceutically acceptable salt thereof or a solvate thereof.

2. A compound as claimed in claim 1 wherein A is absent.

3. A compound as claimed in claim 1 or 2 wherein n is 1 or 2.

4. A compound as claimed in claim 1, 2 or 3 wherein R³ is hydrogen.

5. A compound as claimed in claim 1, 2, 3 or 4 wherein R¹ is NR¹³C(O)R¹⁴; wherein R¹³ and R¹⁴ are as defined in claim 1.

6. A compound as claimed in claim 1, 2, 3 or 4 wherein R¹ is optionally substituted aryl or optionally substituted heteroaryl, wherein the optional substituents are as recited in claim 1.

7. A compound as claimed in claim 1, 2, 3 or 4 wherein R¹ is optionally substituted heterocyclyl.

8. A compound as claimed in any one of the preceding claims wherein R² is phenyl optionally substituted by halo or GF₃.

9. A compound as claimed in any one of the preceding claims wherein X is NR⁶S(O)₂R⁷; wherein R⁶ and R⁷ are as defined in claim 1.

10. A compound as claimed in any one of the preceding claims wherein X is S(O)₂NR⁴R⁵; wherein R⁴ and R⁵ are as defined in claim 1.

11. A process for preparing a compound as claimed in claim 1, the process comprising:
a. when R¹ is an N-linked optionally substituted heterocycle, reacting a compound of formula (II): 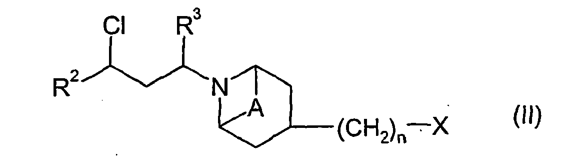 wherein R², R³, n, A and X are as defined in claim 1, with a compound R¹H (wherein the H is on a heterocycle ring nitrogen atom) wherein R¹ is as defined above, in the presence of a suitable base, in a suitable solvent and optionally in the presence of sodium iodide;
b. when R³ is hydrogen, coupling a compound of formula (III): 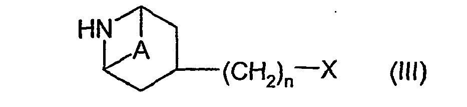 wherein n, A and X are as defined in claim 1, with a compound of formula (IV): 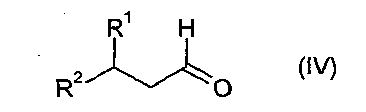 wherein R¹ and R² are as defined in claim 1, in the presence of NaBH(OAc)₃ in a suitable solvent at room temperature;
c. when R³ is hydrogen, coupling a compound of formula (III): 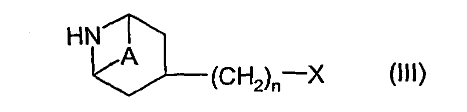 wherein n, A and X are as defined in claim 1, with a compound of formula (V): 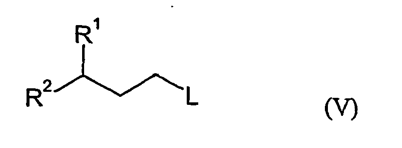 wherein R¹ and R² are as defined in claim 1 and L is a leaving group; in the presence of a base, in a suitable solvent at a temperature from 60°C up to the boiling point of the solvent;
d. when X is S(O)₂NR₄R₅, reacting a compound: 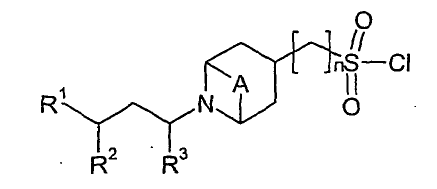 wherein R¹, R², R³, A and n are as defined in claim 1, with NHR⁴R⁵, wherein R⁴ and R⁵ are as defined in claim 1, in the presence of a suitable base and in the presence of a suitable solvent; or,
e. when X is NR⁶S(O)₂NR⁷, reacting a compound: 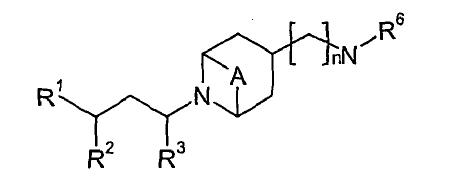
wherein R¹, R², R³, A and n are as defined in claim 1, with R⁷S(O)₂Cl, in the presence of a suitable base and in the presence of a suitable solvent.

12. A pharmaceutical composition which comprises a compound as claimed in claim 1, or a pharmaceutically acceptable salt thereof or solvate thereof, and a pharmaceutically acceptable adjuvant, diluent or carrier.

13. A compound as claimed in claim 1, or a pharmaceutically acceptable salt thereof or solvate thereof, for use as a medicament.

14. A compound as claimed in claim 1, or a pharmaceutically acceptable salt thereof or solvate thereof, in the manufacture of a medicament for use in therapy.

15. Use of a compound as claimed in claim 1, or a pharmaceutical acceptable salt thereof, for the manufacture of a medicament for treating a CCR5 mediated disease state.

## Patentansprüche

1. Verbindung der Formel (I): 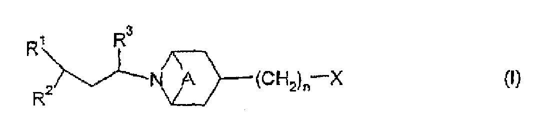 worin:
A fehlt oder für (CH₂)₂ steht;
R¹ für C(O)NR¹⁰R¹¹, C(O)₂R¹², NR¹³C(O)R¹⁴, NR¹⁵C (O) NR¹⁶R¹⁷, NR¹⁸C(O)₂R¹⁹, Heterocyclyl (beispielsweise Piperidin, Piperazin, Pyrrolidin oder Azetidin), Aryl, Cycloalkyl oder Heteroaryl steht;
R¹⁰, R¹³, R¹⁵, R¹⁶ und R¹⁸ für Wasserstoff oder C₁₋₆-Alkyl stehen;
R¹¹, R¹², R¹⁴, R¹⁷ und R¹⁹ für C₁₋₈-Alkyl (gegebenenfalls substituiert durch Halogen, Hydroxy, C₁₋₆-Alkoxy, C₁₋₆-Halogenalkoxy, C₃₋₆-Cycloalkyl (gegebenenfalls substituiert durch Halogen), C₅₋₆-Cycloalkenyl, S(C₁₋₄-Alkyl), S(O)(C₁₋₄-Alkyl), S(O)₂(C₁₋₄-Alkyl), Heteroaryl, Aryl, Herteroaryloxy oder Aryloxy), Aryl, Heteroaryl, C₃₋₇-Cycloalkyl (gegebenenfalls substituiert durch Halogen oder C₁₋₄-Alkyl), an einen Phenylring anelliertes C₄₋₇-Cycloalkyl, C₅₋₇-Cycloalkenyl oder Heterocyclyl (selbst gegebenenfalls substituiert durch Oxo, C(O)(C₁₋₆-Alkyl), S (O)ₖ(C₁₋₄-Alkyl), Halogen oder C₁₋₄-Alkyl) steht oder R¹¹, R¹² R¹⁴ und
R¹⁷ auch für Wasserstoff stehen können; oder R¹⁰ und R¹¹ und/oder R¹⁶ und R¹⁷ gemeinsam einen 4-, 5- oder 6-gliedrigen Ring bilden können, der gegebenenfalls ein Stickstoff-, Sauerstoff- oder Schwefelatom enthält und gegebenenfalls durch C₁₋₆-Alkyl, S(O)₁(C₁₋₆-Alkyl) oder C(O)(C₁₋₆-Alkyl) substituiert ist;
R² für Phenyl, Heteroaryl oder C₃₋₇-Cycloalkyl steht;
R³ für H oder C₁₋₄-Alkyl steht;
X für S(O)₂NR⁴R⁵ oder NR⁶S(O)₂R⁷ steht;
R⁷ für Aryl, Heteroaryl, C₁₋₆-Alkyl, C₃₋₇-Cycloalkyl, Heterocyclyl oder NR⁸R⁹ steht, wobei NR⁸R⁹ zu einem 4-, 5- oder 6-gliedrigen Ring cyclisiert sein kann, der gegebenenfalls ein Stickstoff-, Sauerstoff- oder Schwefelatom enthält und gegebenenfalls durch C₁₋₆-Alkyl, S(O)ₚ(C₁₋₆-Alkyl) oder C(O)(C₁₋₆-Alkyl) substituiert ist;
R⁴ und R⁸ für Aryl, Heteroaryl, C₁₋₆-Alkyl (gegebenenfalls substituiert durch Hydroxy oder C₁₋₆-Alkoxy), C₃₋₇-Cycloalkyl oder Heterocyclyl stehen;
R⁵, R⁶ und R⁹ unabhängig voneinander für Wasserstoff oder C₁₋₆-Alkyl stehen;
n für 1, 2 oder 3 steht;
Aryl-, Phenyl- und Heteroarylgruppen unabhängig voneinander gegebenenfalls ein- oder mehrfach durch Halogen, Cyano, Nitro, Hydroxy, OC(O)NR²⁰R²¹, NR²²R²³, NR²⁴C(O)R²⁵, NR²⁶C(O)NR²⁷R²⁸, S (O)₂NR²⁹R³⁰, NR³¹S(O)₂R³², C(O)NR³³R³⁴, CO₂R³⁶, NR³⁷CO₂R³⁸, S(O)_{q}R³⁹, OS (O)₂R⁴⁹, C₁₋₆-Alkyl (gegebenenfalls einfach substituiert durch S(O)₂R⁵⁰ oder C(O)NR⁵¹R⁵²), C₂₋₆-Alkenyl, C₂₋₆-Alkinyl, C₃₋₁₀-Cycloalkyl, C₁₋₆-Halogenalkyl, C₁₋₆-Alkoxy-C₁₋₆-alkyl, C₂₋₆-Alkoxy, C₁₋₆-Halogenalkoxy, Phenyl, Phenyl-C₁₋₄-alkyl, Phenoxy, Phenylthio, Phenyl-S(O), Phenyl-S(O)₂, Phenyl-C₁₋₄-alkoxy, Heteroaryl, Meteroaryl-C₁₋₄-alkyl, Heteroaryloxy oder Heteroaryl-C₁₋₄-alkoxy substituiert sind; wobei jede der unmittelbar vorstehenden Phenyl- und Heteroarylgruppierungen gegebenenfalls durch Halogen, Hydroxy, Nitro, S(C₁₋₄-Alkyl) , S (O) (C₁₋₄-Alkyl), S (O)₂(C₁₋₄-Alkyl) , S(O)₂NH₂, S(O)₂NH(C₁₋₄-Alkyl), S(O)₂N(C₁₋₄-Alkyl)₂, Cyano, C₁₋₄-Alkyl, C₁₋₄-Alkoxy, C (O) NH₂, C(O) NH (C₁₋₄-Alkyl), C(O)N(C₁₋₄-Alkyl)₂, CO₂H, CO₂(C₁₋₄-Alkyl), NHC(O) (C₁₋₄-Alkyl) NHS (O)₂(C₁₋₄-Alkyl), CF₃ oder OCF₃ substituiert ist;
sofern nicht anders vermerkt, Heterocyclyl gegebenenfalls durch C₁₋₆-Alkyl [gegebenenfalls substituiert durch Phenyl {welches gegebenenfalls selbst durch Halogen, C₁₋₄-Alkyl, C₂₋₄-Alkoxy, Cyano, Nitro, CF₃, OCF₃, (C₁₋₄-Alkyl)C(O)NH, S(O)₂NH₂, C₁₋₄-Alkylthio, S(O) (C₁₋₄-Alkyl) oder S (O)₂(C₁₋₄-Alkyl) substituiert ist} oder Heteroaryl {welches gegebenenfalls selbst durch Halogen, C₁₋₄-Alkyl, C₁₋₄-Alkoxy, Cyano, Nitro, CF₃, (C₁₋₄-Alkyl)C(O) NH, S (O)₂NH₂, C₁₋₄-Alkylthio, S(O) (C₁₋₄-Alkyl) oder S(O)₂(C₁₋₄-Alkyl) substituiert ist}], Phenyl {gegebenenfalls substituiert durch Halogen, C₁₋₄-Alkyl, C₁₋₄-Alkoxy, Cyano, Nitro, CF₃, OCF₃, (C₃₋₄-Alkyl)C(O)NH, S(O)₂NH₂, C₁₋₄-Alkylthio, S(O)(C₁₋₄-Alkyl) oder S (O)₂(C₁₋₄-Alkyl)}, Heteroaryl {gegebenenfalls substituiert durch Halogen, C₁₋₄-Alkyl, C₁₋₄-Alkoxy, Cyano, Nitro, CF₃, (C₁₋₄-Alkyl) C (O) NH, S (O)₂NH₂, C₁₋₄-Alkylthio, S (O) (C₁₋₄-Alkyl) oder S(O)₂(C₁₋₄-Alkyl)}, S(O)₂NR⁴⁰R⁴¹, C(O)R⁴², C(O)₂(C₁₋₆-Alkyl) (wie tert.-Butoxyacarbonyl), C(O)₂(Phenyl(C₁₋₆-alkyl)) (wie Benzyloxycarbonyl), C(O)NHR⁴³, S(O)₂R⁴⁴, NHS(O)₂NHR⁴⁵, NHC(O) R⁴⁶, NHC(O)NHR⁴⁷ oder NHS(O)₂R⁴⁸ substituiert ist, mit der Maßgabe, daß keiner der letzten vier Substituenten an einen Ringstickstoff gebunden ist;
k, 1, p und q unabhängig voneinander für 0, 1 oder 2 stehen;
R²⁰, R²², R²⁴, R²⁶, R²⁷ R²⁹, R³¹, R³³, R³⁷, R⁴⁰ und R⁵¹ unabhängig voneinander für Wasserstoff oder C₁₋₆-Alkyl stehen;
R²¹, R²³, R²⁵, R²⁸, R³⁰, R³², R³⁴, R³⁶, R³⁸, R³⁹, R⁴¹, R⁴² R⁴³, R⁴⁴, R⁴⁵, R⁴⁶, R⁴⁷, R⁴⁸, R⁴⁹, R⁵⁰ und R⁵² unabhängig voneinander für Wasserstoff, C₁₋₆-Alkyl (gegebenenfalls substituiert durch Halogen, Hydroxy, C₁₋₆-Alkoxy, C₁₋₆-Halogenalkoxy, C₃₋₆-Cycloalkyl, C₅₋₆-Cycloalkenyl, S(C₁₋₄-Alkyl) S (O) (C₁₋₄-Alkyl), S(O)₂(C₁₋₄Alkyl), Heteroaryl, Phenyl, Heteroaryl oder Phenyloxy), C₃₋₇-Cycloalkyl, Phenyl oder Heteroaryl stehen; wobei jede der unmittelbar vorstehenden Phenyl- und Heteroarylgruppierungen gegebenenfalls durch Halogen, Hydroxy, Nitro, S(C₁₋₄-Alkyl), S (O) (C₁₋₄-Alkyl), S (O)₂(C₁₋₄-Alkyl), S(O)₂NH₂, S (O)₂NH (C₁₋₄-Alkyl), S(O)₂N (C₁₋₄-Alkyl)₂, Cyano, C₁₋₄-Alkyl, C₁₋₄-Alkoxy, C(O)NH₂, C(O)NH(C₁₋₄-Alkyl), C(O)N(C₁₋₄-Alkyl)₂, CO₂H, CO₂(C₁₋₄-Alkyl), NHC (O) (C₁₋₄-Alkyl) , NHS (O)₂ (C₁₋₄-Alkyl) C (O) (C₁₋₄-Alkyl), CF₃ oder OCF₃ substituiert ist;
R²¹, R²³, R²⁵, R²⁸, R³⁰, R³⁴, R³⁶, R⁴¹, R⁴², R⁴³, R⁴⁵, R⁴⁶ R⁴⁷ und R⁵² zusätzlich auch für Wasserstoff stehen können;
oder ein pharmazeutisch annehmbares Salz davon oder ein Solvat davon.

2. Verbindung nach Anspruch 1, in der A fehlt.

3. Verbindung nach Anspruch 1, oder 2, in der n für 1 oder 2 steht.

4. Verbindung nach Anspruch 1, 2 oder 3, in der R³ für Wasserstoff steht.

5. Verbindung nach Anspruch 1, 2, 3 oder 4, in der R¹ für NR¹³C(O)R¹⁴ steht, wobei R¹³ und R¹⁴ die in Anspruch 1 angegebene Bedeutung besitzen.

6. Verbindung nach Anspruch 1, 2, 3 oder 4, in der R¹ für gegebenenfalls substituiertes Aryl oder gegebenenfalls substituiertes Heteroaryl steht, wobei die fakultativen Substituenten den Angaben in Anspruch 1 entsprechen.

7. Verbindung nach Anspruch 1, 2, 3 oder 4, in der R¹ für gegebenenfalls substituiertes Heterocyclyl steht.

8. Verbindung nach einem der vorhergehenden Ansprüche, in der R² für gegebenenfalls durch Halogen oder CF₃ substituiertes Phenyl steht.

9. Verbindung nach einem der vorhergehenden Ansprüche, in der X für NR⁶S(O)₂R⁷ steht, wobei R⁶ und R⁷ die in Anspruch 1 angegebene Bedeutung besitzen.

10. Verbindung nach einem der vorhergehenden Ansprüche, in der X für S(O)₂NR⁴R⁵ steht, wobei R⁴ und R⁵ die in Anspruch 1 angegebene Bedeutung besitzen.

11. Verfahren zur Herstellung einer Verbindung nach Anspruch 1, bei dem man:
a. wenn R¹ für einen N-verknüpften gegebenenfalls substituierten Heterocyclus steht, eine Verbindung der Formel (II): 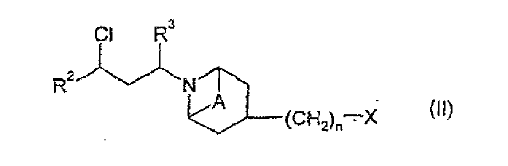 worin R², R³, n, A und X die in Anspruch 1 angegebene Bedeutung besitzen, in Gegenwart einer geeigneten Base in einem geeigneten Lösungsmittel und gegebenenfalls in Gegenwart von Natriumiodid mit einer Verbindung R¹H (worin sich das H an einem Ringstickstoffatom eines Heterocyclus befindet), worin R¹ die oben angegebene Bedeutung besitzt, umsetzt;
b. wenn R³ für Wasserstoff steht, eine Verbindung der Formel (III): 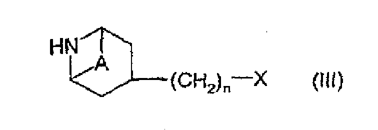 worin n, A und X die in Anspruch 1 angegebene Bedeutung besitzen, in Gegenwart von NaBH(OAc)₃ in einem geeigneten Lösungsmittel bei Raumtemperatur mit einer Verbindung der Formel (IV): 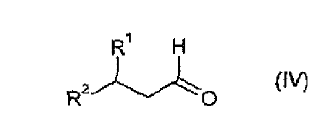 worin R¹ und R² die in Anspruch 1 angegebene Bedeutung besitzen, kuppelt;
c. wenn R³ für Wasserstoff steht, eine Verbindung der Formel (III) : 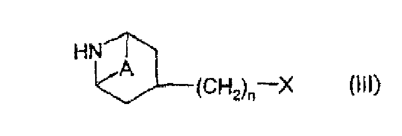 worin n, A und X die in Anspruch 1 angegebene Bedeutung besitzen, in Gegenwart einer Base in einem geeigneten Lösungsmittel bei einer Temperatur von 60°C bis zum Siedepunkt des Lösungsmittels mit einer Verbindung der Formel (V) : 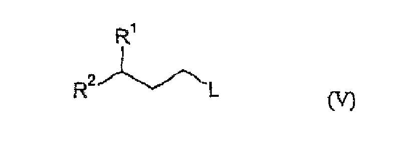 worin R¹ und R² die in Anspruch 1 angegebene Bedeutung besitzen und L für eine Abgangsgruppe steht, kuppelt;
d. wenn X für S(O)₂NR⁴R⁵ steht, eine Verbindung: 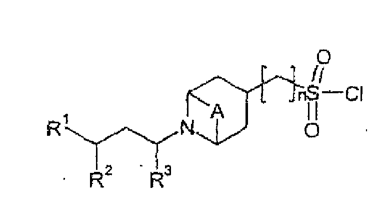 worin R¹, R², R³, A und n die in Anspruch 1 angegebene Bedeutung besitzen, in Gegenwart einer geeigneten Base und in Gegenwart eines geeigneten Lösungsmittels mit NHR⁴R⁵, worin worin R⁴ und R⁵ die in Anspruch 1 angegebene Bedeutung besitzen, umsetzt; oder
e. wenn X für NR⁶S(O)₂NR⁷ steht, eine Verbindung: 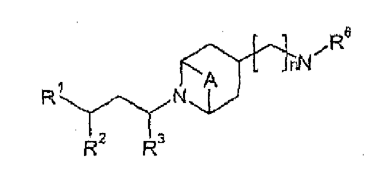
worin R¹, R², R³, A und n die in Anspruch 1 angegebene Bedeutung besitzen, in Gegenwart einer geeigneten Base und in Gegenwart eines geeigneten Lösungsmittels mit R⁷S(O)₂Cl umsetzt.

12. Pharmazeutische zusammensetzung, die eine Verbindung nach Anspruch 1 oder ein pharmazeutisch annehmbares Salz davon oder ein Solvat davon und einen pharmazeutisch annehmbaren Hilfsstoff, ein pharmazeutisch annehmbares Verdünnungsmittel oder einen pharmazeutisch annehmbaren Träger enthält.

13. Verbindung nach Anspruch 1 oder ein pharmazeutisch annehmbares Salz davon oder Solvat davon zur Verwendung als Arzneimittel.

14. Verwendung einer Verbindung nach Anspruch 1 oder eines pharmazeutisch annehmbaren Salzes davon oder Solvats davon bei der Herstellung eines Arzneimittels zur Verwendung bei der Therapie.

15. Verwendung einer Verbindung nach Anspruch 1 oder eines pharmazeutisch annehmbaren Salzes davon zur Herstellung eines Arzneimittels zur Behandlung eines durch CCR5 vermittelten Krankheitszustands.

## Revendications

1. Composé de formule (I) : 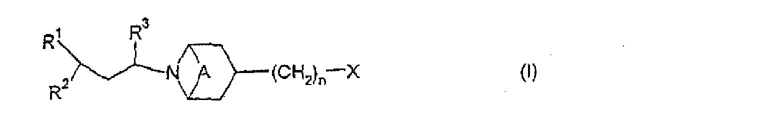 dans laquelle :
A est absent ou est (CH₂)₂ ;
R¹ est C(O)NR¹⁰R¹¹, C(O)₂R¹², NR¹³C(O)R¹⁴, NR¹⁵C(O)NR¹⁶R¹⁷, NR¹⁸C(O)₂R¹⁹, hétérocyclyle (par exemple pipéridine, pipérazine, pyrrolidine ou azétidine), aryle, cycloalkyle ou hétéroaryle ;
R¹⁰, R¹³ , R¹⁵ , R¹⁶ et R¹⁸ sont hydrogène ou C₁₋₆ alkyle ;
R¹¹, R¹², R¹⁴, R¹⁷ et R¹⁹ sont C₁₋₈-alkyle (éventuellement substitué par halogéno, hydroxy, C₁₋₆-alcoxy, C₁₋₆-halogénoalcoxy, C₃₋₆-cycloalkyle (éventuellement substitué par halogéno), C₅₋₆-cycloalcényle, S(C₁₋₄-alkyle), S(O)(C₁₋₄-alkyle), S(O)₂(C₁₋₄-alkyle), hétéroaryle, aryle, hétéroaryloxy ou aryloxy), aryle, hétéroaryle, C₃₋₇-₇-cycloalkyle (éventuellement substitué par halogéno ou C₁₋₄alkyle), C₄₋₇-cycloalkyle condensé sur un cycle phényle, C₅₋₇-cycloalcényle, ou hétérocyclyle (éventuellement substitué lui-même par oxo, C (O) (C₂₋₆-alkyle) S (O)ₖ(C₁₋₆-alkyle), halogéno ou C₁₋₄-alkyle); ou R¹¹, R¹², R¹⁴ et R¹⁷ peuvent également être hydrogène ;
ou R¹⁰ et R¹¹, et/ou R¹⁶ et R¹⁷ peuvent se joindre pour former un cycle à 4, 5 ou 6 chaînons qui inclut éventuellement un atome d'azote, d'oxygène ou de soufre, ledit cycle étant éventuellement substitué par C₁₋₆-alkyle, S (O)₁(C₁₋₆-alkyle) ou C(O) (C₁₋₆-alkyle) ;
R² est phényle, hétéroaryle ou C₃₋₇-cycloalkyle ;
R³ est H ou C₁₋₄ alkyle ;
X est S(O)₂NR⁴R⁵ ou NR⁶S(O)₂R⁷;
R⁷ est aryle, hétéroaryle, C₁₋₆-alkyle, C₃₋₇-cycloalkyle, hétérocyclyle ou NR⁸R⁹ où NR⁸R⁹ peut être cyclisé pour former un cycle à 4, 5 ou 6 chaînons qui inclut éventuellement un atome d'azote, d'oxygène ou de soufre, ledit cycle étant éventuellement substitué par C₁₋₆alkyle, S(O)ₚ(C₁₋₆ alkyle) ou C(O)(C₁₋₆-alkyle)
R⁴ et R⁸ sont aryle, hétéroaryle, C₁₋₆-alkyle (éventuellement substitué par hydroxy ou C₁₋₆-alcoxy), C₃₋₇-cycloalkyle ou hétérocyclyle ;
R⁵, R⁶ et R⁹ sont, indépendamment, hydrogène ou C₁₋₆-alkyle ;
n est 1, 2 ou 3 ;
les motifs aryle, phényle et hétéroaryle sont indépendamment éventuellement substitués par un ou plusieurs éléments parmi halogéno, cyano, nitro, hydroxy, OC(O)NR²⁰R²¹, NR²²R²³, NR²⁴C (O) R²⁵, NR²⁶C(O)NR²⁷R²⁸, S(O)₂NR²⁹R³⁰, NR³¹S(O)₂R³², C(O)NR³³R³⁴, CO₂R³⁶, NR³⁷CO²R³⁸, S(O)_{q}R³⁹, OS(O)₂R⁴⁹, C₁₋₆-alkyle (éventuellement mono-substitué par S(O)₂R⁵⁰ ou C(O)NR⁵¹R⁵²), C₂₋₆alcényle, C₂₋₆-alcynyle, C₃₋₁₀-cycloalkyle, C₁₋₆-halogénoalkyle, C₁₋₆-alcoxy (C₁₋₆)alkyle, C₁₋₆-alcoxy, C₁₋₆-halogénoalcoxy, phényle, phényl(C₁₋₄)alkyle, phénoxy, phénylthio, phénylS(O), phénylS(O)₂, phényl (C₁₋₄) alcoxy, hétéroaryle, hétéroaryl(C₁₋₄)alkyle, hétéroaryloxy ou hétéroaryl(C₁₋₄)alcoxy; où l'un quelconque des motifs phényle et hétéroaryle immédiatement ci-dessus sont éventuellement substitués par halogéno, hydroxy, nitro, S(C₁₋₄-alkyle), S(O)(C₁₋₄-alkyle), S(O)₂(C₁₋₄-alkyle), S (O)₂NH₂, S(O)₂NH(C₁₋₄-alkyle), S(O)₂N(C₁₋₄-alkyle)₂, cyano, C₁₋₄-alkyle, C₁₋₄-alcoxy, C(O)NH₂, C(O)NH(C₁₋₄-alkyle), C(O)N(C₁₋₄-alkyle)₂, CO₂H, CO₂(C₁₋₄-alkyle), NHC(O) (C₁₋₄-alkyle), NHS(O)₂(C₁₋₄-alkyle), CF₃ ou OCF₃ ;
sauf indication contraire hétérocyclyle est éventuellement substitué par C₁₋₆-alkyle [éventuellement substitué par phényle {qui est lui-même éventuellement substitué par halogéno, C₁₋₄-alkyle, C₁₋₄-alcoxy, cyano, nitro, CF₃, OCF₃, (C₁₋₄-alkyl)C(O)NH, S(O)₂NH₂, C₁₋₄-alkylthio, S(O) (C₁₋₄-alkyle) ou S(O)₂(C₁₋₄-alkyle)} ou hétéroaryle {qui est lui-même éventuellement substitué par halogéno, C₁₋₄-alkyle, C₁₋₄-alcoxy, cyano, nitro, CF₃, (C₁₋₄-alkyl)C(O)NH, S (O)₂NH₂, C₁₋₄-alkylthio, S(O) (C₁₋₄-alkyle) ou S (O)₂(C₁₋₄-alkyle)}], phényle {éventuellement substitué par halogéno, C₁₋₄ - alkyle, C₁₋₄-alcoxy, cyano, nitro, CF₃, OCF₃, (C₁₋₄ alkyl)C(O)NH, S(O)₂NH₂, C₁₋₄-alkylthio, S(O)(C₁₋₄-alkyle) ou S(O)₂(C₁₋₄ alkyle)}, hétéroaryle {éventuellement substitué par halogéno, C₁₋₄-alkyle, C₁₋₄-alcoxy, cyano, nitro, CF₃, (C₁₋₄-alkyl)C(O)NH, S(O)₂NH₂, C₁₋₄-alkylthio, S(O) (C₁₋₄-alkyle)}, S(O)₂NR⁴⁰R⁴¹, C(O)R⁴², C(O)2 (C₁₋₆-alkyle) (tel que tert-butoxycarbonyle), C(O)₂(phényl (C₁₋₂-alkyle)) (tel que benzyloxycaxbonyle), C(O)NHR⁴³, S (O)₂R⁴⁴, NHS (O)₂NHR⁴⁵, NHC (O) R⁴⁶, NHC (O) NHR⁴⁷ ou NHS(O)₂R⁴⁸, à condition qu'aucun de ces quatre derniers substituants ne soit lié à un azote du cycle;
k, 1, p et q sont, indépendamment, 0, 1 ou 2;
R²⁰, R²², R²⁴, R²⁶, R²⁷, R²⁹, R³¹, R³³, R³⁷, R⁴⁰ et R⁵¹ sont, indépendamment, hydrogène ou C₁₋₆ alkyle ;
R²¹, R²³, R²⁵, R²⁸, R³⁰, R³², R³⁴, R³⁶, R³⁸, R³⁹, R⁴¹, R⁴², R⁴³, R⁴⁴, R⁴⁵, R⁴⁶, R⁴⁷, R⁴⁸, R⁴⁹, R⁵⁰ et R⁵² sont, indépendamment, C₁₋₆-alkyle (éventuellement substitué par halogéno, hydroxy, C₁₋₆-alcoxy, C₁₋₆-halogénoalcoxy, C₃₋₆-cycloalkyle, C₅₋₆-cycloalcényle, S(C₁₋₄-alkyle), S(O)(C₁₋₄-alkyle), S(O)₂(C₁₋₄-alkyle), hétéroaryle, phényle, hétéroaryloxy ou phényloxy), C₃₋₇-cycloalkyle, phényle ou hétéroaryle ; où l'un quelconque des motifs phényle et hétéroaryle immédiatement ci-dessus sont éventuellement substitués par halogéno, hydroxy, nitro, S(C₁₋₄-alkyle), S(O)(C₁₋₄-alkyle), S (O)₂(C₁₋₄-alkyle), S (O)₂NH₂, S (O)₂NH (C₁₋₄-alkyle) , S-(O)₂N(C₁₋₄-alkyle)₂, cyano, C₁₋₄-alkyle, C₁₋₄-alcoxy, C (O)NH₂, C(O)NH(C₁₋₄-alkyle), C(O)N(C₁₋₄-alkyle)₂, CO₂H, CO₂(C₁₋₄-alkyle), NHC(O) (C₁₋₄-alkyle), NHS (O)₂(C₁₋₄-alkyle), C(O) (C₁₋₄-alkyle), CF₃ ou OCF₃ ;
R²¹, R²3, R²⁵, R²⁸, R³⁰*,* R³⁴, R³⁵, R³⁶, R⁴¹*,* R⁴², R⁴³, R⁴⁵, R⁴⁶, R⁴⁷ et R⁵² peuvent en outre être hydrogène;
ou un sel pharmaceutiquement acceptable de celui-ci ou un solvate de celui-ci.

2. Composé selon la revendication 1, **caractérisé en ce que** A est absent.

3. Composé selon la revendication 1 ou 2, **caractérisé en ce que** n est 1 ou 2.

4. Composé selon la revendication 1, 2 ou 3, **caractérisé en ce que** R³ est hydrogène.

5. Composé selon la revendication 1, 2, 3 ou 4, **caractérisé en ce que** R¹ est NR¹³C(O)R¹⁴ ; où R¹³ et R¹⁴ sont tels que définis dans la revendication 1.

6. Composé selon la revendication 1, 2, 3 ou 4, **caractérisé en ce que** R¹ est aryle éventuellement substitué ou hétéroaryle éventuellement substitué, où les substituants éventuels sont tels que cités dans la revendication 1.

7. Composé selon la revendication 1, 2, 3 ou 4, **caractérisé en ce que** R¹ est hétérocyclyle éventuellement substitué.

8. Composé selon l'une quelconque des revendications précédentes, **caractérisé en ce que** R² est phényle éventuellement substitué par halogéno ou CF₃.

9. Composé selon l'une quelconque des revendications précédentes, **caractérisé en ce que** X est NR⁶S(O)2R⁷; où R⁶ et R⁷ sont tels que définis dans la revendication 1.

10. Composé selon l'une quelconque des revendications précédentes, **caractérisé en ce que** X est S(O)₂NR⁴R⁵; où R⁴ et R⁵ sont tels que définis dans la revendication 1.

11. Procédé de préparation d'un composé selon la revendication 1, le procédé comprenant :
a. lorsque R¹ est un hétérocycle éventuellement substitué lié par N, la réaction d'un composé de formule (II): 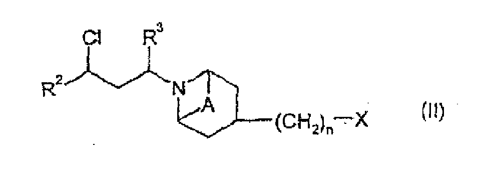 dans laquelle R², R³, n, A et X sont tels que définis dans la revendication 1, avec un composé R¹H (où le H est un atome d'azote du cycle hétérocyclique) dans lequel R¹ est tel que défini ci-dessus, en présence d'une base convenable, dans un solvant convenable et éventuellement en présence d'iodure de sodium ;
b. lorsque R³ est hydrogène, le couplage d'un composé de formule (III) : 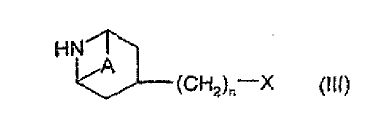 dans laquelle n, A et X sont tels que définis dans la revendication 1, avec un composé de formule (IV) _{:} 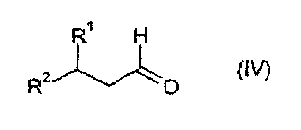 dans laquelle R¹ et R² sont tels que définis dans la revendication 1, en présence de NaBH(OAc)₃ dans un solvant convenable, à température ambiante ;
c. lorsque R³ est hydrogène, le couplage d'un composé de formule (III) : 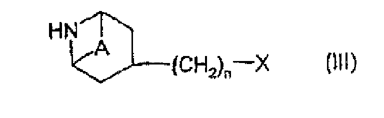 dans laquelle n, A et X sont tels que définis dans la revendication 1, avec un composé de formule (V) : 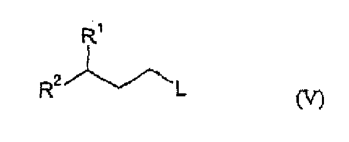 dans laquelle R¹ et R² sont tels que définis dans la revendication 1 et L est un groupement partant ; en présence d'une base, dans un solvant convenable, à une température de 60°C jusqu'au point d'ébullition du solvant ;
d. lorsque X est S(O)₂NR⁴R⁵, la réaction d'un composé : 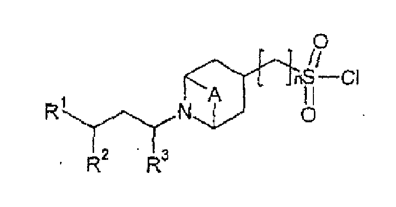 dans lequel R¹, R², R³, A et n sont tels que définis dans la revendication 1, avec NHR⁴R⁵, où R⁴ et R⁵ sont tels que définis dans la revendication 1, en présence d'une base convenable et en présence d'un solvant convenable ; ou,
e. lorsque X est NR⁶S(O)₂NR⁷, la réaction d'un composé : 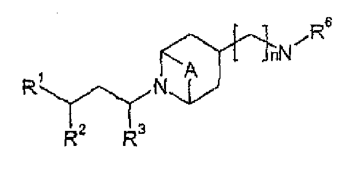
dans lequel R¹, R², R³, A et n sont tels que définis dans la revendication 1, avec R⁷S (O)₂Cl, en présence d'une base convenable et en présence d'un solvant convenable.

12. Composition pharmaceutique, **caractérisée en ce qu'**elle comprend un composé selon la revendication 1, ou un sel ou un solvate pharmaceutiquement acceptable de celui-ci, et un adjuvant, un diluant ou un support pharmaceutiquement acceptable.

13. Composé selon la revendication 1, ou un sel ou un solvate pharmaceutiquement acceptable de celui-ci, destiné à être utilisé comme médicament.

14. Composé selon la revendication 1, ou un sel ou un solvate pharmaceutiquement acceptable de celui-ci, dans la fabrication d'un médicament destiné à être utilisé en thérapie.

15. Utilisation d'un composé selon la revendication 1, ou d'un sel pharmaceutiquement acceptable de celui-ci, pour la fabrication d'un médicament destiné au traitement d'un état pathologique médié par le CCR5.
